# EUROPEAN PATENT APPLICATION

(11) **EP 2 500 424 A1**
(43) Date of publication of application: **19.09.2012**
(21) Application number: 10829686.4
(22) Date of filing: 04.11.2010
(51) Int. Cl.: C12N 11/00, C12M 1/34, C12M 1/42, C12N 11/08, C12N 13/00, C12Q 1/02, G01N 33/48

(54) **SUBSTRATE WITH PHOTO-CONTROLLABLE CELL ADHESION PROPERTY, METHOD FOR ANALYZING AND FRACTIONATING CELLS, AND DEVICE FOR ANALYSIS AND FRACTIONATION OF CELLS**

(30) Priority: 13.11.2009 JP 2009259398; 17.02.2010 JP 2010031936
(71) Applicant: Hitachi High-Technologies Corporation, Tokyo 105-8717 (JP)
(72) Inventor: SUGIYAMA, Hisashi, Hitachinaka-shi Ibaraki 312-8504 (JP); TAKAHASHI, Satoshi, Hitachinaka-shi Ibaraki 312-8504 (JP); UCHIDA, Kenko, Kokubunji-shi Tokyo 185-8601 (JP); OZAWA, Satoshi, Kokubunji-shi Tokyo 185-8601 (JP)
(74) Representative: Strehl Schübel-Hopf & Partner
(86) International application number: PCT/JP2010/006476
(87) International publication number: WO 2011/058721

(57) **Abstract**

When cells are analyzed, fractionated, and incubated while keeping the cells alive, real-time operations can be performed more easily and the cells can be incubated while removing unnecessary cells from the incubated cells to purify the cells being incubated. Furthermore, desired cells are separated through analysis from the incubated cells, and the purity, recovery, and viability of the cells are heightened. Use is made of a substrate having photo-controllable cell adhesion properties, the substrate comprising a transparent base and, formed thereon, a film of a material which has photo-controllable cell adhesion properties and has been obtained by bonding a cell-adhesive material to a cell-non-adhesive material through photo-dissociable groups. Cell images are detected and analyzed to obtain information about the location of desired cells. On the basis of the information, a space is formed between cells and the material having photo-controllable cell adhesion properties is cut, by means of second light irradiation. Meanwhile, by means of first light irradiation, the surface of the substrate is changed from a cell-adhesive surface to a cell-non-adhesive surface, thereby separating the cell(s) from the substrate. Thus, cells can be analyzed and fractionated while keeping the cells alive.

## Description

### Technical Field

The present invention relates to the field of regenerative medicine and stem-cell research, and particularly relates to a technique for analysis and fractionation/culture of cells.

### Background Art

In the field of regenerative medicine, the preparation of somatic cells involves identifying and isolating very small numbers of somatic stem cells or progenitor cells contained in somatic cells and culturing the resultant. Attempts for preparing somatic cells have been also made by using iPS cells or ES cells as a source for somatic cells: inducing differentiation from them into somatic stem cells or somatic cells and culturing the resultant. However, the iPS cells or ES cells are not homogeneous and cells differentiated from them are also not homogeneous. Various cells occur. Cells or tissues used for regenerative medicine are required to provide homogeneous somatic cells, to contain somatic stem cells, and to be free of cancer cells or cancer stem cells or pluripotent stem cells such as iPS cells and ES cells.

Thus, techniques for analyzing, fractionating, and culturing cells become increasingly important in the field of regenerative medicine. To fractionate cells of multiple types, an analysis technique is necessary for distinguishing them. In addition, without fractionating the cells of the distinguished types into cells of single types, the molecular biological properties or cell biological properties of cells of single types cannot be analyzed. The induction of differentiation can be strictly controlled without satisfying these requirements. Further, a technique is required for removing unnecessary cells when a differentiation induction efficiency of 100% is not achieved.

Devices for analyzing cells alive include a well-known light microscope, a fluorescence microscope for observing fluorescently labeled cells, and a fluorometric imaging device; however, these devices cannot fractionate cells. On the other hand, devices for fractionating cells alive include an device for the separate collection of desired cells by the antigen-antibody reaction between an antigen on the cell surface and an antibody added to magnetic beads; however, this device cannot analyze cells and has a problem in the purity, recovery rate, and the like thereof. Devices for fractionating cells also include a laser microdissection device; however, it is mainly used for isolation from a dead cell section embedded in paraffin.

Devices for analyzing and fractionating cells alive include a flow cytometer and a sorting device which are well known. These devices are each an device by which cells are analyzed and distinguished by exposing individual cells in a sample stream imposed on a sheath stream to laser light and observing scattered light or fluorescence, followed by giving charges to droplets containing individual cells based on the information for fractionation by applying the electric field. A multicolored laser light can be irradiated to analyze many fluorescent markers; however, this requires complicated fluorescence correction and optical axis adjustment. When trypsin treatment or the like is performed to separate a mass of cells into individual cells in advance, the cells are not a little damaged. In addition, although the sorted cells have high purity and a high recovery rate, they have problems including that the viability thereof is reduced by impact during sorting. For treatment using these devices, cells must be once taken out of a culture substrate.

Techniques for analyzing, fractionating, and culturing cells alive include a method as described in JP 3975266. This technique is associated with a device for using a cell culture substrate having a photoresponsive material, film-formed, whose physical properties are changed by light irradiation, distinguishing between cultured cells with a minitor, locating desired cells, subjecting the desired cell position to light pattern irradiation, and detaching the desired cells from the culture substrate. As the "photoresponsive material whose physical properties are changed by light irradiation" described here, one is cited which has a function by which cells are detached from the culture substrate by the isomerization of the structure thereof by light irradiation to change the polarizability and hydophilic-hydrophbic property thereof; particularly, changes in these physical properties are considered to be preferably reversible.

### Citation List

### Patent Literature

Patent Literature 1: JP 3975266
Patent Literature 2: JP 3472723
Patent Literature 3: JP 2004-170930 A
Patent Literature 4: JP 2008-167695 A

### Non Patent Literature

Non Patent Literature 1: Kazuhiko Ishihara, Seitai Zairyo (Biocompatible Material) 18 (1): 33 (2000).
Non Patent Literature 2: Y. Arima et al., J. Meter. Chem., 17: 4079 (2007).
Non Patent Literature 3: Y. Arima et al., Biomaterials 28: 3074 (2007).
Non Patent Literature 4: M. N. Yousaf et al., PNAS 98 (11): 5992 (2001).
Non Patent Literature 5: Toshiaki Furuta, Kogaku (Optics) 34 (4): 213 (2005)
Non Patent Literature 6: J. Edahiro et al., Biomacromolecules, 6(2): 970 (2005).
Non Patent Literature 7: J. Nakanishi et al., Analytical Sciences, 24: 67 (2008).

### Summary of Invention

### Technical Problem

As the photoresponsive material whose physical properties are changed by light irradiation described in the above JP 3975266, the material whose structure is reversibly changed by light is difficult to be 100% one of the two isomers because cell adhesion has reduced selectivity. The material responsive to light of a long wavelength as described in Examples will be changed in adhesion, for example, by responding to exciting light for fluorescent observation. In addition, while the technique can detach cells from a culture substrate, it does not contemplate the detachment of the adhesion between cells. Thus, it will wholly detach an isolated cell or a cell mass present in the culture substrate, leaving a problem that a cell mass consisting of a plurality of types of cells adhering to each other cannot be fractionated to single cells.

In view of the foregoing prior art, the present invention is directed to provide a photo-controllable cell-adhesive substrate for analyzing, fractionating, and culturing cells alive and a method for analyzing and fractionating cells and a device therefor.

An object of the present invention is to allow more simple operation in real time and culture while removing unnecessary cells from cultured cells for purification in analyzing, fractionating, and culturing the cells alive and to analyze and fractionate desired cells from the cultured cells to increase the purity, recovery rate, and viability of the cells as compared to before.

### Solution to Problem

To solve the above prior art problems, the present invention has adopted the following means.

That is, the photo-controllable cell-adhesive substrate of the present invention is obtained by film-forming a photo-controllable cell-adhesive material in which a cell-adhesive material is bonded to a cell-non-adhesive material through a photo-dissociable group, on a base.

In the photo-controllable cell-adhesive substrate of the present invention, light irradiation causes the bond dissociation of the photo-dissociable group to produce the separation of the cell-adhesive material to leave the cell-non-adhesive material.

In the photo-controllable cell-adhesive substrate of the present invention, light irradiation causes the bond dissociation of the photo-dissociable group to irreversibly change the surface of the irradiated portion thereof from that of the cell-adhesive material to that of the cell-non-adhesive material.

The method for analyzing and fractionating cells according to the present invention comprises the following steps.

A step of seeding and culturing cells on a photo-controllable cell-adhesive substrate obtained by film-forming a photo-controllable cell-adhesive material in which a cell-adhesive material is bonded to a cell-non-adhesive material through a photo-dissociable group, on a base, or a photo-controllable cell-adhesive substrate, wherein light irradiation causes the bond dissociation of the photo-dissociable group to produce the separation of the cell-adhesive material to leave the cell-non-adhesive material, or a photo-controllable cell-adhesive substrate, wherein light irradiation causes the bond dissociation of the photo-dissociable group to irreversibly change the surface of the irradiated portion thereof from that of the cell-adhesive material to that of the cell-non-adhesive material.

A step of detaching and recovering desired cells from the substrate by first light irradiation on desired cellular regions.

The method for analyzing and fractionating cells according to the present invention also comprises the following step.

A step of providing cell-adhesive regions and a cell-non-adhesive region by first light irradiation on a photo-controllable cell-adhesive substrate obtained by film-forming a photo-controllable cell-adhesive material in which a cell-adhesive material is bonded to a cell-non-adhesive material through a photo-dissociable group, on a base, or a photo-controllable cell-adhesive substrate, wherein light irradiation causes the bond dissociation of the photo-dissociable group to produce the separation of the cell-adhesive material to leave the cell-non-adhesive material, or a photo-controllable cell-adhesive substrate, wherein light irradiation causes the bond dissociation of the photo-dissociable group to irreversibly change the surface of the irradiated portion thereof from that of the cell-adhesive material to that of the cell-non-adhesive material.

The device for analyzing and fractionating cells according to the present invention comprises a photo-controllable cell-adhesive substrate obtained by film-forming a photo-controllable cell-adhesive material in which a cell-adhesive material is bonded to a cell-non-adhesive material through a photo-dissociable group, on a base, or a photo-controllable cell-adhesive substrate, wherein light irradiation causes the bond dissociation of the photo-dissociable group to produce the separation of the cell-adhesive material to leave the cell-non-adhesive material, or a photo-controllable cell-adhesive substrate, wherein light irradiation causes the bond dissociation of the photo-dissociable group to irreversibly change the surface of the irradiated portion thereof from that of the cell-adhesive material to that of the cell-non-adhesive material, and a first light irradiation means for subjecting the photo-controllable cell-adhesive material on the base to photoreaction.

### Advantageous Effect of Invention

In analyzing, fractionating, and culturing cells alive, operations can be more simply made in real time and culture can be performed while removing unnecessary cells from cultured cells for purification. Desired cells can also be analyzed and fractionated from the cultured cells to increase the purity, recovery rate, and viability of the cells.

### Brief Description of Drawings

[Fig. 1] Fig. 1 is a diagram showing a method for analyzing cells during culture and fractionating desired cells (Examples 1, 2, 5, and 6).
[Fig. 2] Fig. 2 is a diagram showing a method for analyzing individually separated cells and fractionating desired cells (Examples 3, 7, and 9).
[Fig. 3] Fig. 3 is a diagram showing a method for analyzing individually separated cells and fractionating desired cells (Examples 4 and 8).
[Fig. 4] Fig. 4 is a diagram showing a device for performing the method for analyzing and fractionating cells using the photo-controllable cell-adhesive substrate according to the present invention (Example 10).
[Fig. 5] Fig. 5 is a device for performing the method for analyzing and fractionating cells using the photo-controllable cell-adhesive substrate according to the present invention (Example 11).

### Description of Embodiments

Embodiments of the present invention will be described below. However, the present invention is not intended to be limited thereto.

One embodiment of the present invention is a photo-controllable cell-adhesive substrate obtained by film-forming a photo-controllable cell-adhesive material in which a cell-adhesive material is bonded to a cell-non-adhesive material through a photo-dissociable group, on a base. In the photo-controllable cell-adhesive material, light irradiation can cause the bond dissociation of the photo-dissociable group to produce the separation of the cell-adhesive material from the substrate. The bond dissociation may occur between the cell-non-adhesive material and the photo-dissociable group or between the photo-dissociable group and the cell-adhesive material. The light irradiation leaves the cell-non-adhesive material in the substrate. The irreversible photodissociation reaction can efficiently change the irradiated surface from the cell-adhesive one to the cell-non-adhesive one, enabling the enhancement of adhesion selectivity.

Examples of the cell-non-adhesive material include a biocompatible material with a phosphorylcholine group, having a structure similar to that of a cell membrane. The cell-non-adhesive material is, for example, a (meth)acrylic ester polymer with a phosphorylcholine group, represented by general formula (1) below.

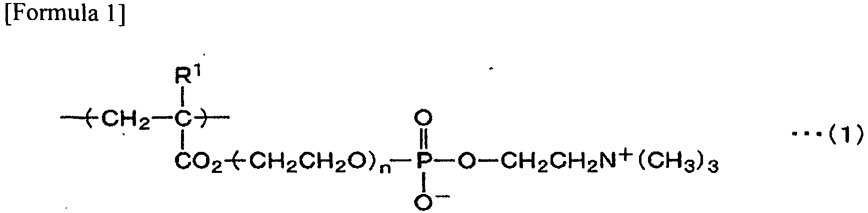

wherein R¹ represents hydrogen or a methyl group and n represents a number of 1 to 20.
A (meth)acrylic ester polymer represented by general formula (2) below may also be used as the cell-non-adhesive material.

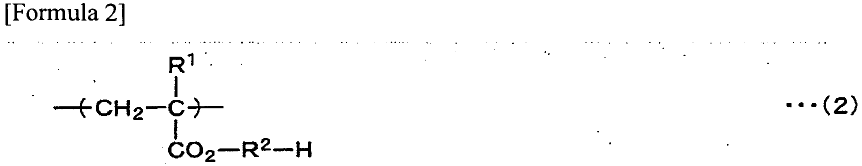

wherein R² represents 1 to 20 alkylene groups or 1 to 20 polyoxyethylene groups.
The cell-non-adhesive material may be a copolymer of (meth)acrylic ester polymers represented by the above general formulas (1) and (2). In addition, the cell-non-adhesive material may use an alkoxysilane represented by general formula (3) below.

[Formula 3] (R³O)₃Si—R²—H (3)

wherein R³ represents hydrogen or an alkyl group.
Examples of the cell-adhesive material include a material having a cell-adhesive group in the terminal end. The cell-adhesive material preferably uses a material comprising general formula (4) below.

[Formula 4] — X (4)

wherein X represents a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, or a nucleic acid group.
The cell-adhesive group X of the general formula (4) can be varied to provide a variation in adhesion to various cells. In addition, the cell-adhesive material encompasses a material in which an extracellular matrix capable of promoting adhesion to cells or an antibody capable of binding to the surface antigen of cells and a protein or the like for causing the antibody to bind thereto binds or adheres to the above general formula (4). Examples of the extracellular matrix include collagens, non-collagenous glycoproteins (fibronectin, vitronectin, laminin, nidogen, tenascin, thrombospondin, von Willebrand, osteopontin, fibrinogen, and the like), elastins, and proteoglycans. Examples of the protein capable of causing the antibody to bind include avidin/biotin, protein A, and protein G.

The wavelength of the photoreaction of the photo-dissociable group should be 360 nm or more which is non-cytotoxic and a shorter wavelength than the wavelength of incident light for light microscopical observation or exciting light for fluorescent observation. This ensures that a change in adhesion does not occur during cell observation. Examples of the photo-dissociable group include an o-nitrobenzyl group, a hydroxyphenacyl group, and a coumarinylmethyl group. However, a material comprising a coumarinylmethyl skeleton is good in that it has no cytotoxicity and is high in the wavelength of the photoreaction and the photoreaction efficiency thereof. Particularly, a material comprising a coumarinylmethyl skeleton of general formula (5) below can be suitably used.

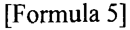

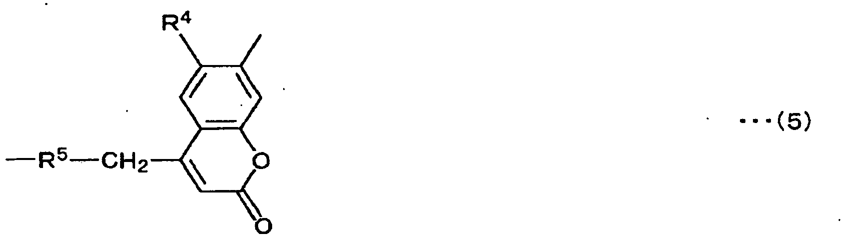

wherein R⁴ represents hydrogen or an alkoxy group and R⁵ is divalent and represents O, CO, CO₂, OCO₂, NHCO₂, NH, SO₃, or (OPO(OH))_{1 to 3}OPO₂.
The bonding of the photo-dissociable group and the cell-adhesive material forms a structure in which a cell-adhesive group represented by the general formula (4) directly or indirectly bonds to a photo-dissociable group represented by the general formula (5) at position 7 of the coumarin skeleton or at the position of R⁵. Photodissociation occurs at the position of R⁵. For example, the bonding at position 7 of the coumarin skeleton forms a structure in which the bonding is via a divalent linking group R⁶, as represented by general formula (6) below.

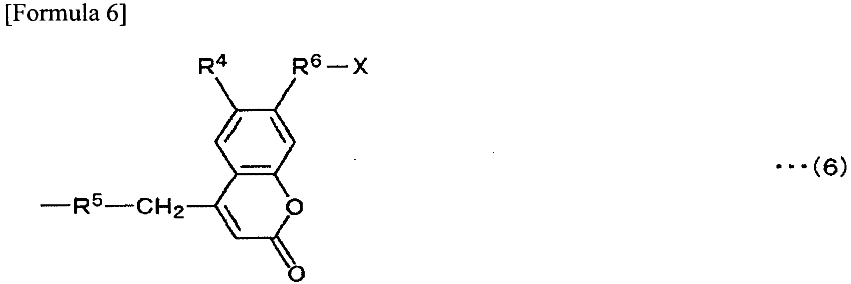

The divalent linking group R⁶ may use O(CH₂)ₘ, O(CH₂CH₂O)ₘ, OCO(CH₂)ₘ, OCOCH₂O(CH₂CH₂O)ₘ (where m is an integer of 0 to 20), or the like; however, R⁶ is only intended to serve the purpose of bonding the photo-dissociable group to the cell-adhesive group. If the photo-dissociable group is turned end for end, the photodissociation can occur between the photo-dissociable group and the cell-adhesive group. Examples thereof can include general formula (7) below representing a structure in which the bonding occurs at the position of R⁵.

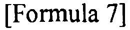

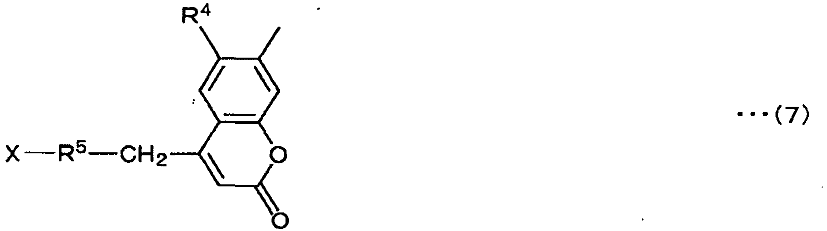

The structure in which the cell-adhesive material and the photo-dissociable group are bonded are directly or indirectly bonded to the cell-non-adhesive material.

For example, the structure may be made in the form of a (meth)acrylic ester polymer such as general formula (8), (9), or (10) below and incorporated into the cell-non-adhesive material.

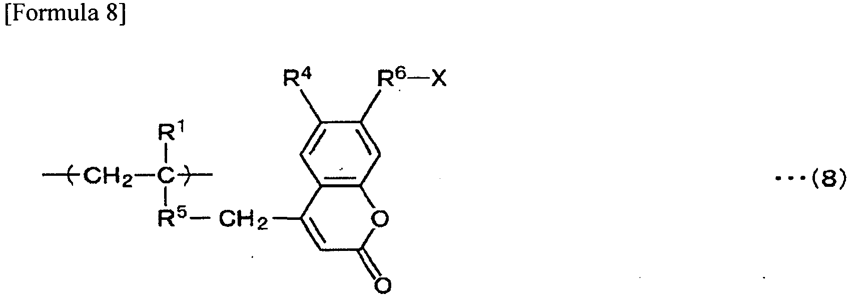

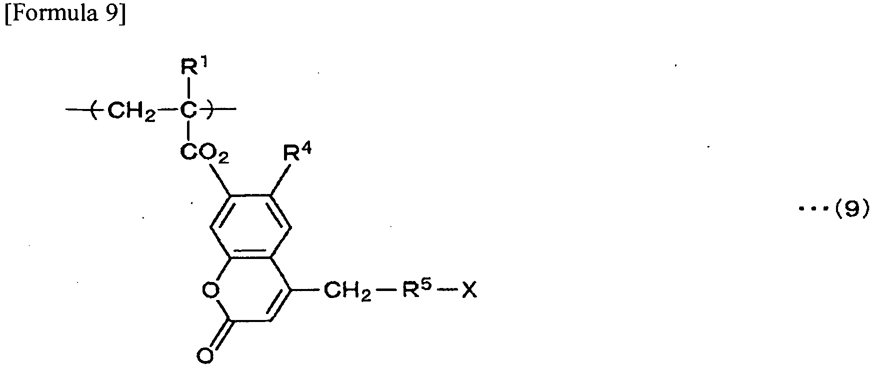

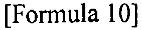

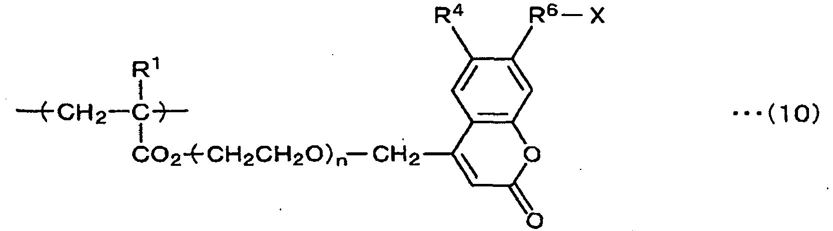

Examples of the material in which the structure having the cell-adhesive material and the photo-dissociable group bonded is bonded to the cell-non-adhesive material can include a copolymer of (meth)acrylic ester polymers represented by the general formulas (1) and (8), the general formulas (1) and (9), the general formulas (1) and (10), the general formulas (1), (2), and (8), the general formulas (1), (2), and (9), or the general formulas (1), (2), and (10). The copolymer can be made to change the ratio of the cell-adhesive material and the cell-non-adhesive material, which will provide a variation in adhesion to various cells. The copolymerization of a (meth)acrylic ester containing an alkoxysilane in the side chain with each of these polymers can increase adhesion to the base.

Although these systems take the form of copolymers, they may be in the form of homopolymers. For example, the system may use the general formula (10) alone, and may also take the form of an alkoxysilane represented by general formula (11) or (12) below.

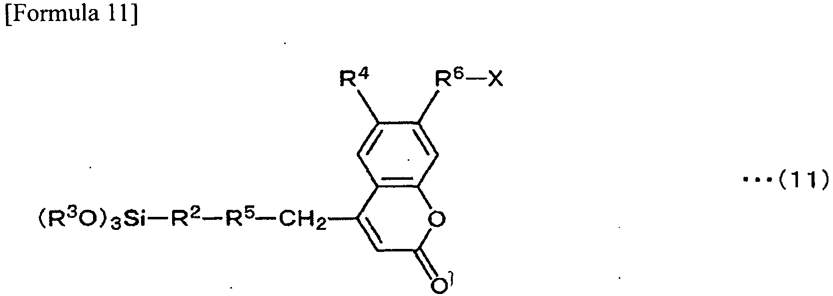

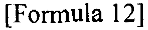

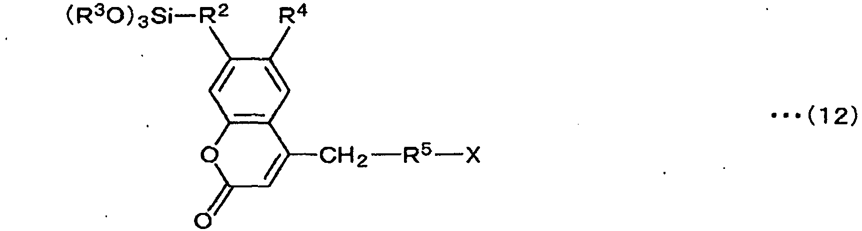

These cell-adhesive materials again include a material to which an extracellular matrix capable of promoting adhesion to cells or an antibody capable of binding to the surface antigen of cells and a protein or the like for causing the antibody to bind thereto is bound or adheres.

The base for film-forming the photo-controllable cell-adhesive material thereon may use a transparent plastic culture vessel or the like; however, a glass culture vessel may be preferably used in view of optical performance and durability.

The method for analyzing and fractionating cells using the photo-controllable cell-adhesive substrate will now be described in detail based on figures.

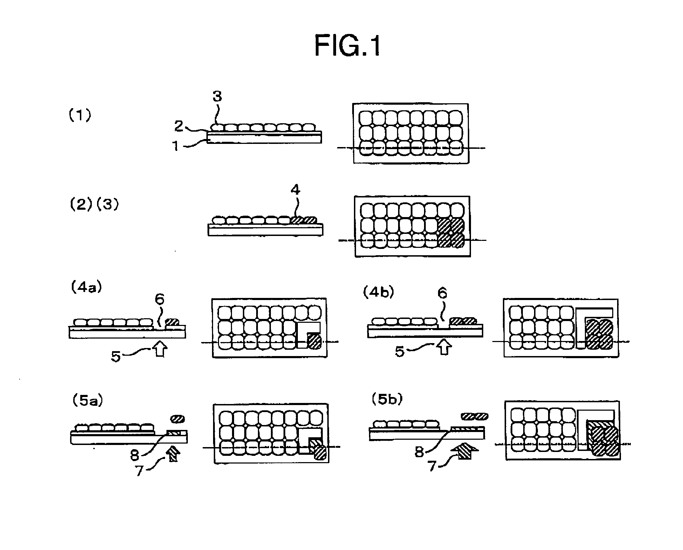

Fig. 1 illustrates one aspect of the method for analyzing and fractionating cells according to the present invention. Each left side shows the cross section by a dashed line. (1) The cells 3 are seeded and cultured on the photo-controllable cell-adhesive material 2 film-formed on a glass culture vessel (transparent base) 1. (2) Cell images are detected by microscopic observation (including the observation of transmission images, phase contrast images, differential interference images, and the like), fluorescent observation, scattered light observation, Raman light observation, or the like; a characteristic amount of cells are extracted; and then (3) the positional information of desired cells (regardless of necessity) is obtained. Fluorescent marker labeling or the like may be performed before or after culture. In (4a), for example, the cells 4 are unnecessary cells, and the periphery of the side of the cell 4 and a photo-controllable cell-adhesive material 6 in the boundary between the cell 3 and the cell 4 are cut by the second light irradiation 5. Laser light can be preferably used for the second light irradiation 5. (5a) When the area of the cell 4 is wide, first light irradiation 7 is performed on the region 8 of the cell 4 to change the photo-controllable cell-adhesive material into a cell-non-adhesive one, and the remaining cell 4 is detached from a glass culture vessel 1 and recovered together with the culture solution. When the area of the cell 4 is small, all cells 4 and the photo-controllable cell-adhesive material 8 may be cut/destructed by the second light irradiation 5. Thereafter, the culture is continued, and the cells 3 may continue to be cultured while sequentially removing unnecessary cells 4 for purification.

(4b) is a case where it is desired to fractionate/isolate the cells 4 for analysis (a case where the cells 4 are necessary). The periphery of the side of the cell 3 in the boundary between the cell 3 and the cell 4 and the photo-controllable cell-adhesive material 6 are cut by the second light irradiation 5.

The second light irradiation 5 can preferably use laser light. Thereafter, (5b) the first light irradiation 7 is performed on the region 8 of the cell 4 to change the photo-controllable cell-adhesive material into a cell-non-adhesive one, and the cell 4 is detached from the glass culture vessel 1 and recovered together with the culture solution.

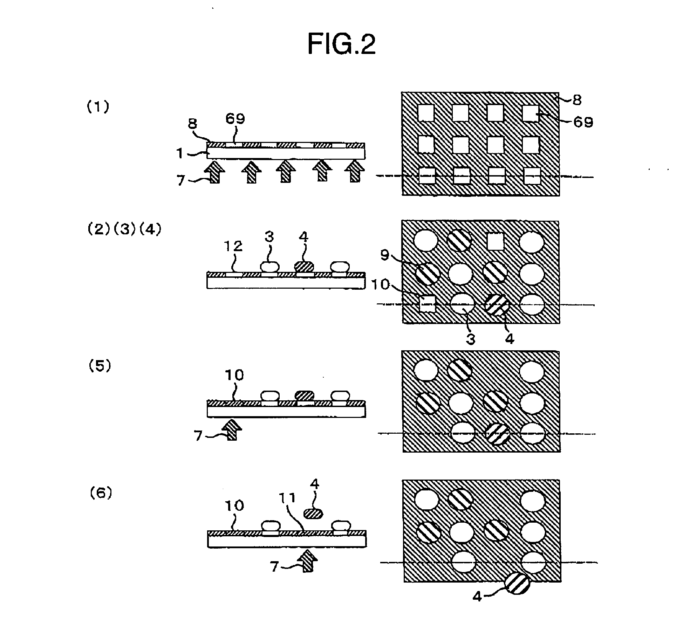

Fig. 2 illustrates another aspect of the method for analyzing and fractionating cells according to the present invention. Each left side shows the cross section by a dashed line. The first light irradiation 7 is performed on the photo-controllable cell-adhesive material 2 film-formed on a glass culture vessel 1 as shown in the figure to provide the cell-adhesive regions 69 and the cell-non-adhesive region 8. The cell-adhesive regions 69 and the cell-non-adhesive region 8 may each be set in any pattern; however, the cell-adhesive regions 69 were here arranged in a lattice form as areas of single cells. In other words, the first light irradiation 7 was performed so that the cell-adhesive regions 69 were arranged in a lattice form.

(2) An already cultured cell mass is then separated into individual cells by treatment with trypsin or the like and seeded. (3) Cell images are detected by microscopic observation (including the observation of transmission images, phase contrast images, differential interference images, and the like), fluorescent observation, scattered light observation, Raman light observation, or the like; a characteristic amount of cells are extracted; and then (4) the positional information of desired cells (regardless of necessity) is obtained. Fluorescent marker labeling or the like may be performed before or after culture. Here, it is assumed that in addition to cells 3, other cells (for example, cells 4 and cells 9) are present. (5) When cells do not adhere to all of the addresses (cell-adhesive regions 69), the appropriate addresses are subjected to the first light irradiation 7 and thereby made cell-non-adhesive (reference symbol 10 in the figure). (6) Areas 11 of desired cells, cells 4 here, are then subjected to the first light irradiation 7, and the cells 4 are detached from the glass culture vessel 1 and recovered together with the culture solution. The step (6) can be sequentially repeated to fractionate and isolate the cells 3 and 9.

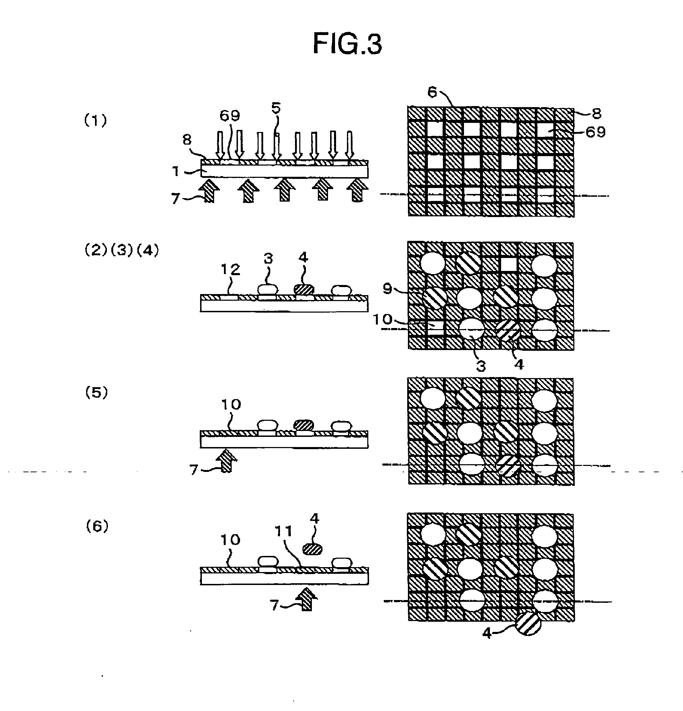

Fig. 3 illustrates still another aspect of the method for analyzing and fractionating cells according to the present invention. Each left side shows the cross section by a dashed line. The second light irradiation (for example, with laser light) 5 is performed on the photo-controllable cell-adhesive material 2 film-formed on the glass culture vessel 1 in columns and rows at predetermined intervals to cut the adjacent photo-controllable cell-adhesive material 2 (section line: 6). The first light irradiation 7 was performed on predetermined positions to provide the cell-adhesive regions 69 and the cell-non-adhesive regions 8.

The cell-adhesive regions 69 and the cell-non-adhesive region 8 may each be set in any pattern; however, the cell-adhesive regions 69 were here arranged in a lattice form as areas of single cells. (2) An already cultured cell mass is then separated into individual cells by treatment with trypsin or the like and seeded. (3) Cell images are detected by microscopic observation (including the observation of transmission images, phase contrast images, differential interference images, and the like), fluorescent observation, scattered light observation, Raman light observation, or the like; a characteristic amount of cells are extracted; and then (4) the positional information of desired cells (regardless of necessity) is obtained. Fluorescent marker labeling or the like may be performed before or after culture. Here, it is assumed that in addition to cells 3, cells 4 and cells 9 are present.

(5) When cells do not adhere to all of the cell-adhesive regions (addresses) 69, the appropriate addresses 10 are subjected to the first light irradiation 7 and thereby made cell-non-adhesive. (6) Areas 11 of desired cells, cells 4 here, are subjected to the first light irradiation 7, and the cells 4 are detached from the glass culture vessel 1 and recovered together with the culture solution. The step (6) can be sequentially repeated to fractionate and isolate the cells 3 and 9. The difference with Fig. 2 lies in that when a fibrous or membranous material such as an extracellular matrix or feeder cells is present on the upper layer of the cell-adhesive material, the cell-adhesive material is cut between the regions with the second light irradiation 5 because it is not cut into the regions with only the first light irradiation 7.

The device for performing the method for analyzing and fractionating cells using the photo-controllable cell-adhesive substrate (for example, a photo-controllable cell-adhesive material film-formed on a transparent base) of the present invention at least comprises (1), (2), (3), (4), (6), and (7) below:
(1) the photo-controllable cell-adhesive substrate;
(2) a stage on which the substrate is placed;
(3) an optical detection means for obtaining cell images;
(4) a means for obtaining positional information from cell images;
(5) a second light irradiation means for cutting or destructing between cells and a photo-controllable cell-adhesive material;
(6) a first light irradiation means for subjecting the photo-controllable cell-adhesive material on the base to photoreaction; and
(7) a means for controlling the motion of each means.
The optical detection means for obtaining cell images as described in (3) above may use a known optical system. In obtaining cell images, light is irradiated which has a wavelength not affecting the photo-dissociable group of the photo-controllable cell-adhesive material. For example, using a lamp or LED providing broad emission spectra as a light source, light is irradiated through a short-wavelength cut filter for at least removing light of not more than the photoreaction wavelength to detect transmitted light, reflected light, or the like using a 2-dimensional sensor such as CCD. In the case of fluorescent images from cells, light of the absorption wavelength range of a desired fluorochrome, having the range of a wavelength longer than the photoreaction wavelength is irradiated as an exciting light by dispersion with a bandpass interference filter or the like. Laser light of the above wavelength range may also be used. The fluorescence is detected with a 2-dimensional sensor such as CCD through a wavelength filter such as an exciting light cut filter or a fluorescence wavelength transmission filter (a bandpass interference filter or the like). If the exciting light is sharply restricted and a 2-dimensional-scanning mode is adopted, the fluorescent image can also be measured using a photomultiplier tube. Measurement can be made by switching a plurality of wavelength filters to provide fluorescent images of a plurality of fluorescence wavelengths, enabling application to a plurality of fluorophores. If it is passed through a dispersive element such as a prism or a diffracting grating and detected with a line sensor or the like, a finer wavelength spectrum image can also be obtained.

The second irradiation means described in (5) above uses an infrared laser or an ultraviolet laser as the light source and can be performed by laser scanning based on the positional information described in (4) above. The laser scanning uses an XY deflector and light irradiation is performed on desired positions. Light pattern irradiation can also be performed by one operation through a photomask reflecting the positional information described in (4) above. In that case, an optical system for condensing laser light on the substrate through a spatial light modulation device to avoid the preparation of a fixed photomask every experiment is preferable as a pattern generator. The spatial light modulation device can use a reflex or transmissive spatial light modulation device. The reflex spatial light modulation device can use a digital mirror device, and the transmissive spatial light modulation device can use a liquid crystal spatial light modulation device. Here, the laser wavelength usable in the digital mirror device or the liquid crystal spatial light modulation device is mainly in the range from visible light to near-infrared light. Thus, a near infrared laser, which can strongly absorb water, can be used as a laser source. When it is desired to set the wavelength region within the ultraviolet region, visible to near infrared laser light is passed through a spatial light modulation device and then passed through a wavelength conversion device such as a nonlinear crystal or a ferroelectric crystal to use a second harmonic or a third harmonic, which has a 1/2 or 1/3 wavelength, respectively.

The first light irradiation means described in (6) above uses a wavelength of the photoreaction of the photo-controllable cell-adhesive material as a light source. A lamp, LED, or the like providing a broad emission spectrum is used, and a wavelength of 360 nm or less and, in some cases, not less than the fluorescence excitation wavelength is cut by a wavelength filter; or a laser of a photoreaction wavelength can be used. It is also possible that light scanning is performed using an XY deflector based on the positional information described in (4) above for light irradiation on desired positions, or light pattern irradiation is performed by one operation through a photomask reflecting the positional information described in (4) above. In that case, an optical system for making light condensation on the substrate through a spatial light modulation device to avoid the preparation of a fixed photomask every experiment is preferable as a pattern generator. The spatial light modulation device can use a reflex or transmissive spatial light modulation device. The reflex spatial light modulation device can use a digital mirror device, and the transmissive spatial light modulation device can use a liquid crystal spatial light modulation device.

The optical systems described in (3), (5), and (6) above preferably use parts as common as possible.

### Example 1

A ternary copolymer of 20 mole% of a methacrylic acid polymer represented by the general formula (1) (R¹: methyl, n: 1), 50 mole% of a methacrylic acid polymer represented by the general formula (2) (R¹: methyl, R²: butylene), and 30 mole% of a methacrylic acid polymer represented by the general formula (10) (R¹: methyl, R⁶: OCOCH₂OCH₂CH₂OCH₂CH₂ R⁴: Br, X: CH₂CO₂H, n: 1) is film-formed on a glass culture vessel. A cell suspension of human bone-marrow stroma cells and a human fat cell differentiation medium (Cell Applications) is added thereto and cultured at 37°C in a CO₂ incubator. In a stage in which a confluent state of 40% is reached, the glass culture vessel is disposed in the device of the present invention, and microscopic observation is carried out by cutting light of a wavelength of 450 nm or less. Positions of probably abnormal cells are identified by a monitor, and the periphery of within the group of abnormal cells is set to a laser ablation region (see (1), (2), (3), (4a), and (5a) in Fig. 1). Laser light of 1,064 nm or 355 nm is laser-scanned or pattern-irradiated to cut between normal fat cells and abnormal cells and the photo-controllable cell-adhesive material. Thereafter, the scanning of a laser or light for photoreaction or the laser or light pattern irradiation is performed by cutting light of 360 nm or less on the region of an abnormal cell group within the region surrounded by the laser ablation irradiation. The glass culture vessel is taken out of the device, and the abnormal cell group is recovered together with the medium, and the fresh medium is added, followed by returning the resultant to the incubator for the continuation of culture.

This Example is one example; however, various types of cells can be caused to adhere by properly selecting the cell-adhesive group of the photo-controllable cell-adhesive material or properly controlling the ratio of the cell-adhesive material to the cell-non-adhesive material. A photodissociation reaction efficiently and irreversibly changes the cell-adhesive material into the cell-non-adhesive material, showing excellence in the adhesion selectivity between cells and the substrate, and further the cutting of the adhesion between cells and the photo-controllable cell-adhesive material using laser light enables the enhancement of the purity and recovery rate of desired cells present in any region in recovering these cells. In addition, it is unnecessary that in culturing cells, the cells be once taken out of the culture substrate and purified as for a flow cytometer or a sorting device, and the culture can be performed on the same culture substrate while removing unnecessary cells in real time, simplifying the culture/purification operation. When normal cells contact or mix with abnormal cells, the abnormal cells are spatially separated from the normal cells and an abnormal cell region is compartmentalized. This enables the photodissociation reaction to be conducted to be limited to the abnormal cell region, enabling the selective detachment of the abnormal cells. The cells once detached do not re-adhere because the original place is irreversibly changed into a cell-non-adhesive one, enabling the effective removal of the abnormal cells. In addition, an electrical stimulus and an impact as for a flow cytometer or a sorting device are not present, and the control of the photoreaction wavelength, the light wavelength for microscopic observation, and the exciting light wavelength for fluorescent observation can reduce phototoxicity to cells; thus, the viability of cells can be increased. Further, cells are arranged on a 2-dimensional plane, almost simultaneously exposed to light, and subjected to microscopic or fluorescent observation; thus, optical axis adjustment for 1-dimensionally arranging cells and exposing individual cells to a laser as for the flow cytometer is unnecessary.

### Example 2

After continuing to culture the sample of Example 1, the glass culture vessel is taken out of the incubator. The cells are washed with PBS and treated with a blocking solution for cell surface markers (JRH) for 1 hour. To detect a mesenchymal stem cell marker CD105, a diluted blocking solution for cell surface markers of mouse anti-human CD105 antibody (abcam) is added thereto, which is then reacted at room temperature for 1 hour. After washing with PBS, a diluted blocking solution for cell surface markers of Alexa Fluor 488-labeled anti-mouse IgG antibody (Invitrogen) is added thereto, which is then subjected to reaction under light shielding for 1 hour. After reaction, the solution was replaced with PBS. Then, to detect fat cells, Oil Red O Stain Solution (Sigma) was added thereto, which was then allowed to stand for 1 hour for staining, followed by replacing the solution with PBS. The observation and fractionation of cells were performed as follows. The glass culture vessel is disposed in the device of the present invention. In microscopic observation and fluorescent observation, light of 450 nm or less among light source wavelengths is cut to avoid photoreaction. The positions of mesenchymal stem cells and fat cells are identified by a monitor by microscopic observation and fluorescent observation to set laser ablation regions within the respective cell groups (see (1), (2), (3), (4b), and (5b) in Fig. 1). Laser light of 1,064 nm or 355 nm is laser-scanned or pattern-irradiated to cut between the mesenchymal stem cells, the fat cells, and other cells and the photo-controllable cell-adhesive material to separate the respective cell group regions. Thereafter, light of a wavelength of around 400 nm containing no light of 360 nm or less for photoreaction is first used on the mesenchymal stem cell region to perform the scanning of a laser or light for photoreaction or the laser or light pattern irradiation. Then, the detached mesenchymal stem cells floating in the medium are recovered together with the medium. Next, after washing and adding the medium, a different region, i.e., the fat cell region, is subjected to the scanning of a laser or light for photoreaction or the laser or light pattern irradiation. Then, the detached fat cells floating in the medium are recovered together with the medium.

Unlike Example 1, this Example involves reversing the region in which the photodissociation reaction is caused to separate desired normal cells with high purity, a high recovery rate, and high viability. This Example also has the same advantages as those of Example 1.

### Example 3

HBSS is added to another glass culture vessel in which culture has been performed as in Example 2, before washing, and a trypsin/EDTA solution is added thereto, which is allowed to stand for several minutes to detach cells from the glass culture vessel. Thereafter, a trypsin neutralizing solution was added thereto to stop the reaction; the cells were recovered in a centrifuging tube by pipetting and centrifuged for several minutes; and the supernatant was removed and a medium was added thereto to make a cell suspension. The addition of mesenchymal stem cells and the staining of fat cells were performed as described in Example 2. Then, an alkoxysilane represented by the general formula (11) (R²: (CH₂CH₂O)₂CH₂CH₂), R³: methyl, R⁴: Br, R⁵: O, R⁶: OCOCH₂O(CH₂CH₂O)₃, X: CH₂NH₂) was film-formed on a glass culture vessel, and, after adding PBS, the vessel was disposed in the device of the present invention. Then, the region other than cell-adhesive regions 70 µm square was subjected to laser or light scanning or laser or light pattern irradiation so that the regions were arranged in a lattice form at a 140 µm pitch, and the reaction product was removed together with PBS and again washed with PBS. The cell suspension was seeded and rocked on the glass culture vessel and then allowed to stand for 30 minutes. To remove non-adhering cells, the vessel was disposed in the device of the present invention after exchanging the medium. The observation and fractionation of cells were performed as follows. In microscopic observation and fluorescent observation, light of 450 nm or less among light source wavelengths is cut to avoid photoreaction. The positions of mesenchymal stem cells, fat cells, other cells, and non-adherence of cells are detected using the color information of each address from the microscopic observation and the fluorescent observation. The address regions of non-adherence of cells in the cell-adhesive regions are subjected to the scanning of a laser or light for photoreaction or the laser or light pattern irradiation; after ensuring that recovered cells will not adhere again, the address regions of mesenchymal stem cells are subjected to the scanning of a laser or light for photoreaction or the laser or light pattern irradiation by cutting light of 360 nm or less; and the detached mesenchymal stem cells are recovered together with the medium. Next, the medium was added thereto; the address regions of fat cells were subjected to the scanning of a laser or light for photoreaction or the laser or light pattern irradiation; and the detached fat cells were recovered together with the medium.

In this Example, in addition to the same advantages as those of Examples 1 and 2, in analyzing cells, individual cells can be caused to adhere to addresses in a lattice form to accelerate the speed of optical detection and analytic fractionation. Such a method enables ready fractionation while observing and analyzing the response and the like of cells to a compound, enabling real time handling.

### Example 4

On a glass culture vessel coated with collagen were seeded 1.0×10⁵ undifferentiated mouse ES cells, to which a medium for ES cells and a differentiation-inducing factor such as a cell growth factor were then added, followed by culture for 7 days. Thereafter, the cells were detached with a 0.25% trypsin/1 mM PBS solution and suspended in the medium for ES cells.

Separately, the same glass culture vessel coated with collagen as that in Example 1 was provided, and, after adding PBS, disposed in the device of the present invention. Then, the photo-controllable cell-adhesive material was cut by scanning laser light of 1,064 nm or 355 nm in a checkerboard pattern so that cell-adhesive regions 20 µm square were arranged in a lattice form at a 40 µm pitch. Then, the region other than the cell-adhesive regions was subjected to laser or light scanning or laser or light pattern irradiation, and the reaction product was removed together with PBS and again washed with PBS.

The cell suspension was seeded and rocked thereon and then allowed to stand for 30 minutes. To remove non-adhering cells, the medium was exchanged, and the observation and fractionation of cells were performed as follows. In microscopic observation, light of 450 nm or less among light source wavelengths was cut to avoid photoreaction. The position of liver cells was detected using the cell image information of each address from the microscopic observation. The address regions of non-adherence of cells in the cell-adhesive regions were subjected to the scanning of a laser or light for photoreaction or the laser or light pattern irradiation; after ensuring that recovered cells would not adhere again, the address regions of liver cells were subjected to the scanning of a laser or light for photoreaction or the laser or light pattern irradiation by cutting light of 360 nm or less; and the detached liver cells were recovered together with the medium.

In addition to having the same advantages as those of Examples 1 and 2, this Example effectively acts on the patterning of the material when a fibrous or membranous material such as an extracellular matrix or feeder cells is used for adhering to cells. When the fibrous or membranous material is present on the upper layer, there is a possibility that the cell-adhesive material is not cut for each region by only the first light irradiation. This is cut between the regions by the second light irradiation.

### Example 5

A ternary copolymer of a methacrylic ester polymer represented by the general formula (1) (R¹: CH₃, n: 1), a methacrylic ester polymer represented by the general formula (2) (R¹: CH₃, R²: butylene), and a methacrylic ester polymer represented by the general formula (8) (R¹: CH₃, R⁴: Br, R⁵: CO₂, R⁶: OCOCH₂CH₂, X: CO₂H) is film-formed on a glass culture vessel (base area: 9.6 cm2). As a model of unnecessary cells are provided 12.3×10⁴ NIH/3T3 cells (mouse fibroblast-derived cell line), which are then suspended in 1.6 mL of a medium specific to the cells (10% calf serum, 90% DMEM). The NIH/3T3 cell suspension is added to the glass culture vessel, which is then cultured in an incubator at 37°C and 5% CO₂ for 1 day. AS a model of necessary cells are provided 24×10⁴ HCT116 cells (human colon cancer-derived cell line), which are then suspended in 1.6 mL of a medium specific to the cells (10% FBS, 90% McCoy's 5a). The medium is removed from the glass culture vessel on which the NIH/3T3 cells are cultured, and the suspension of the HCT116 cells is added to the glass culture vessel, which is then cultured in an incubator at 37°C and 5% CO₂ for 1 day. Separately, both cells are similarly cultured alone, and their phase-contrast images are obtained. Incidentally, the HCT116 cells have a cobblestone cell morphology, and the NIH/3T3 cells have a fusiform cell morphology. The glass culture vessel is disposed in the device of the present invention, and phase microscopy is performed by cutting light of 450 nm or less. Positions of unnecessary cells (i.e., fusiform NIH/3T3 cells) are identified by a monitor, and the periphery of within the group of the unnecessary cells is set to a laser ablation region (see (1), (2), (3), (4a), and (5a) in Fig. 1). Laser light of 1,064 nm or 355 nm is laser-scanned or pattern-irradiated to cut between the necessary cells (i.e., cobblestone HCT116 cells) and the unnecessary cells and the photo-controllable cell-adhesive material. Thereafter, the scanning of a laser or light for photoreaction or the laser or light pattern irradiation is performed by cutting light of 360 nm or less on the region of an unintended cell group within the region surrounded by the laser ablation irradiation. The glass culture vessel is taken out of the device, and the unnecessary cell group is recovered together with the medium, and the fresh medium is added, followed by returning the resultant to the incubator for the continuation of culture. This enables the continuation of culture by removing almost all the unnecessary cells and leaving the necessary cells.

In this Example, the principle of operation was shown using the model cells; however, the same operation can also be performed by replacing necessary cells with normal cells and unnecessary cells with abnormal cells. Specifically, for example, the same application is possible, for example, to the purpose of selection/separation by removing abnormal cells (cells differentiated to unintended cells, undifferentiated cells in which stem cell properties are as-maintained, or the like) and leaving normal cells (cells differentiated as intended, or the like) in studies on differentiation induction in stem cells. Conversely, the same operation can also be performed by replacing necessary cells with abnormal cells and unnecessary cells with the normal cells. Specifically, for example, the same application is also possible, for example, to the purpose of selection/separation and concentration by removing normal cells (unintended cells not becoming cancerous) and leaving abnormal cells (intended cancer cells, or the like) in studies on cancer cells.

The above example has illustrated a case where cells are determined using a cell morphology as a criteria with a phase-contrast microscope as a means for observing cells; however, cells can also be determined using other means. For example, using a fluorescence staining reagent specific for cells, the discrimination between intended cells and unintended cells is also possible by using fluorescent microscopic images.

This Example enables the adherence of various types of cells by properly selecting the cell-adhesive group of the photo-controllable cell-adhesive material or properly controlling the ratio of the cell-adhesive material to the cell-non-adhesive material. A photodissociation reaction efficiently and irreversibly changes the cell-adhesive material into the cell-non-adhesive material, showing excellence in the adhesion selectivity between cells and the substrate, and further the cutting of the adhesion between cells and the photo-controllable cell-adhesive material using laser light enables the enhancement of the purity and recovery rate of desired cells present in any region in recovering these cells. In addition, it is unnecessary that in culturing cells, the cells be once taken out of the culture substrate and purified as for a flow cytometer or a sorting device, and the culture can be performed on the same substrate while removing unnecessary unintended cells in real time, simplifying the culture/purification operation. When intended cells contact or mix with unintended cells, the intended cells are spatially separated from the unintended cells and the cell region is compartmentalized. This enables the photodissociation reaction to be conducted to be limited to the unintended cell region, enabling the selective detachment of the unintended cells. The cells once detached do not re-adhere because the original place is irreversibly changed into a cell-non-adhesive one, enabling the effective removal of the unintended cells. In addition, an electrical stimulus or an impact as for a flow cytometer or a sorting device is not present, and the control of the photoreaction wavelength, the light wavelength for phase microscopy, and the exciting light wavelength for fluorescent observation can reduce phototoxicity to cells; thus, the viability of cells can be increased. Further, cells are arranged on a 2-dimensional plane, almost simultaneously exposed to light, and subjected to phase-contrast microscopic or fluorescent observation; thus, optical axis adjustment for 1-dimensionally arranging cells and exposing individual cells to a laser is unnecessary.

### Example 6

After continuing to culture the sample of Example 5 for 4 days, the glass culture vessel is taken out of the incubator. The cells are washed with PBS and treated with a blocking solution for cell surface markers (JRH) for 1 hour. Stain solutions in which FITC-labeled mouse anti-human HLA-A, B, and C antibodies (BioLegend) and a PE (phycoerythrin)-labeled rat anti-mouse H-2 antibody (BioLegend) for detecting HLA antigen as a marker for human cells and detecting H-2 antigen as a marker for mouse cells, respectively are diluted with a diluted blocking solution for cell surface markers are added thereto, which is then reacted at room temperature for 1 hour and washed with PBS. The observation and fractionation of cells were performed as follows. The glass culture vessel is disposed in the device of the present invention. In microscopic observation and fluorescent observation, light of 450 nm or less among light source wavelengths is cut to avoid photoreaction. The positions of human cells (labeled with FITC, fluorescence wavelength: 520 nm, greenish-orange color) and mouse cells (labeled with PE, fluorescence wavelength: 575 nm, orange color) are identified by a monitor by fluorescent observation and phase-contrast microscopic observation to set laser ablation regions within the respective cell groups (see (1), (2), (3), (4b), and (5b) in Fig. 1). Laser light of 1,064 nm or 355 nm is laser-scanned or pattern-irradiated to cut between the human cells and the mouse cells and the photo-controllable cell-adhesive material to separate the respective cell group regions. Thereafter, light of a wavelength of around 400 nm containing no light of 360 nm or less for photoreaction is first used on the human cell region to perform the scanning of a laser or light for photoreaction or the laser or light pattern irradiation. Then, the detached mesenchymal stem cells floating in the medium are recovered together with the medium. Next, after washing and adding the medium, a different region, i.e., the mouse cell region, is subjected to the scanning of a laser or light for photoreaction or the laser or light pattern irradiation, if necessary. Then, the detached mouse cells floating in the medium are recovered together with the medium.

Unlike Example 5, this Example involves reversing the region in which the photodissociation reaction is caused to separate desired necessary cells with high purity, a high recovery rate, and high viability.

Particular advantages of performing fluorescent observation as in this Example include the advantage that even when a trace of unnecessary cells are mixed, necessary cells and unnecessary cells can each be selectively fluorescently stained, followed by detecting and identifying each cells with high sensitivity by fluorescence detection to select and separate only necessary cells with high accuracy. This Example also enables 2 types or more of necessary cells to be set from a mixture of 3 types or more of cells to sequentially recover them.

In this Example, the principle of operation was shown using the model cells; however, the same operation can also be performed by replacing necessary cells with human cells and unnecessary cells with mouse feeder cells. Specifically, for example, application is possible, for example, to the purpose of removing unnecessary cells (mouse feeder cells) and sequentially selecting and separating a plurality of types of intended cells (human stem cells maintaining pluripotency and various differentiated cells differentiated from human stem cells) in studies on the maintenance of undifferentiation or induction of differentiation of human stem cells. In this case, a cell morphology under a phase-contrast microscope, fluorescent staining using various stem cell markers as indicators, or the like can be adopted to determine the undifferentiated properties of human stem cells. Off course, the model cells used in this Example can each be set according to various purposes as in Example 5. Other advantages of this Example are the same as those of Example 5.

### Example 7

PBS is added to another glass culture vessel in which culture has been performed as in Example 5, before washing, and a trypsin/EDTA solution is added thereto, which is allowed to stand at room temperature for several minutes to detach cells from the glass culture vessel. Thereafter, a trypsin-inhibiting solution was added thereto to stop the reaction; the cells were recovered in a centrifuging tube by pipetting and centrifuged for several minutes; and the supernatant was removed and a medium was added to make a cell suspension. Cell staining using a human cell marker and a mouse cell marker as indicators was carried out as in Example 6. Then, a glass culture vessel on which the same material as that in Example 5 was film-formed was provided, and after adding PBS, disposed in the device of the present invention. Then, the region other than cell-adhesive regions 20 µm square was subjected to laser or light scanning or laser or light pattern irradiation so that the regions were arranged in a lattice form at a 40 µm pitch, and the reaction product was removed together with PBS and again washed with PBS. The cell suspension was seeded and rocked on the glass culture vessel and then allowed to stand for 3 hours. Alternatively, to accelerate adherence, after seeding and rocking, cells were precipitated on the bottom of the glass culture vessel using a plate centrifuge or the like, and then allowed to stand for 30 minutes. To remove non-adhering cells, after exchanging the medium, the vessel was disposed in the device of the present invention. The observation and fractionation of cells were performed as follows. In microscopic observation and fluorescent observation, light of 450 nm or less among light source wavelengths is cut to avoid photoreaction. The positions of human cells, mouse cells, and non-adherence of cells are detected using the color information of each address from the microscopic observation and the fluorescent observation. The address regions of non-adherence of cells in the cell-adhesive regions are subjected to the scanning of a laser or light for photoreaction or the laser or light pattern irradiation; after ensuring that recovered cells will not adhere again, the address regions of human cells are subjected to the scanning of a laser or light for photoreaction or the laser or light pattern irradiation by cutting light of 360 nm or less; and the detached human cells are recovered together with the medium. If necessary, then, the medium was added thereto; the address regions of mouse cells were subjected to the scanning of a laser or light for photoreaction or the laser or light pattern irradiation; and the detached mouse cells were recovered together with the medium.

In this Example, in addition to the same advantages as those of Examples 5 and 6, in analyzing cells, individual cells can be caused to adhere to addresses in a lattice form to accelerate the speed of optical detection and analytic fractionation. Such a method has the advantage of enabling ready fractionation while observing and analyzing the response and the like of cells to a compound, enabling real time handling.

### Example 8

15.4×10⁴ Colo320HSR cells was suspended in a medium for the cells (10% FBS, 90% RPMI1640), which was then seeded on a glass culture vessel coated with collagen and cultured for 7 days. Thereafter, they were detached using a 0.25% trypsin PBS solution and, after terminating the reaction, suspended in the same fresh medium.

Separately, the same glass culture vessel coated with collagen as that in Example 5 was provided, and, after adding PBS, disposed in the device of the present invention. Then, the photo-controllable cell-adhesive material was cut by scanning laser light of 1,064 nm or 355 nm in a checkerboard pattern so that cell-adhesive regions 20 µm square were arranged in a lattice form at a 40 µm pitch. Then, the region other than the cell-adhesive regions was subjected to laser or light scanning or laser or light pattern irradiation, and the reaction product was removed together with PBS and again washed with PBS.

The cell suspension was seeded and rocked thereon and then allowed to stand for 3 hours. Alternatively, to accelerate adherence, after seeding and rocking, cells were precipitated on the bottom of the glass culture vessel using a plate centrifuge or the like, and then allowed to stand for 30 minutes. To remove non-adhering cells, the medium was exchanged, and the observation and fractionation of cells were performed as follows. In microscopic observation, light of 450 nm or less among light source wavelengths was cut to avoid photoreaction. The positions of the Colo320HSR cells are detected using cell image information of each address from the microscopic observation. The address regions of non-adherence of cells in the cell-adhesive regions were subjected to the scanning of a laser or light for photoreaction or the laser or light pattern irradiation; after ensuring that recovered cells would not adhere again, the address regions of Colo320HSR cells were subjected to the scanning of a laser or light for photoreaction or the laser or light pattern irradiation by cutting light of 360 nm or less; and the detached Colo320HSR cells were recovered together with the medium.

In addition to having the same advantages as those of Examples 5 and 6, this Example effectively acts on the patterning of the material when a fibrous or membranous material such as an extracellular matrix or feeder cells is used for adhering to cells. When the fibrous or membranous material is present on the upper layer, there is a possibility that the cell-adhesive material is not cut for each region by only the first light irradiation. This is cut between the regions by the second light irradiation.

In this Example, collagen was used as an extracellular matrix, and the Colo320HSR cells were used as a model for cells having low adherence. In this Example, other extracellular matrixes and cells having low adherence can also be similarly preferably used. Extracellular matrixes include fibronectin, laminin, and gelatin in addition to the above collagen, and feeder cells which can be used include mouse fetal fibroblast (MEF) cells, STO cells, 3T3 cells, and SNL cells subjected to growth termination treatment using gamma ray irradiation or antibiotics. Examples of cells requiring an extracellular matrix also include pluripotent stem cells such as ES cells and iPS cells and corneal epithelial stem cells.

### Example 9

PBS is added to another glass culture vessel in which culture has been performed as in Example 7, before washing, and a trypsin/EDTA solution is added thereto, which is allowed to stand at room temperature for several minutes to detach cells from the glass culture vessel. Thereafter, a trypsin neutralizing solution was added thereto to stop the reaction; the cells were recovered in a centrifuging tube by pipetting and centrifuged for several minutes; and the supernatant was removed and a medium was added to make a cell suspension. Fluorescent staining using a human cell marker and a mouse cell marker as indicators was also carried out as in Example 7. Next, a ternary copolymer of a methacrylic ester polymer represented by the general formula (1) (R¹: CH₃, n: 1), a methacrylic ester polymer represented by the general formula (2) (R¹: CH₃, R²: butylene), and a methacrylic ester polymer represented by the general formula (9) (R¹: CH3, R⁴: Br, R⁵-X: OCOCH₂CH₂-CO₂H) is film-formed on a glass culture vessel, and, after adding PBS, disposed in the device of the present invention. Then, the region other than cell-adhesive regions 20 µm square was subjected to laser or light scanning or laser or light pattern irradiation so that the regions were arranged in a lattice form at a 40 µm pitch, and the reaction product was removed together with PBS and again washed with PBS. The cell suspension was seeded and rocked on the glass culture vessel and then allowed to stand for 3 hours. Alternatively, to accelerate adherence, after seeding and rocking, cells were precipitated on the bottom of the glass culture vessel using a plate centrifuge or the like, and then allowed to stand for 30 minutes. To remove non-adhering cells, after exchanging the medium, the vessel was disposed in the device of the present invention. The observation and fractionation of cells were performed as follows. In microscopic observation and fluorescent observation, light of 450 nm or less among light source wavelengths is cut to avoid photoreaction. The positions of human cells, mouse cells, and non-adherence of cells are detected using the color information of each address from the microscopic observation and the fluorescent observation. The address regions of non-adherence of cells in the cell-adhesive regions are subjected to the scanning of a laser or light for photoreaction or the laser or light pattern irradiation; after ensuring that recovered cells will not adhere again, the address regions of human cells are subjected to the scanning of a laser or light for photoreaction or the laser or light pattern irradiation by cutting light of 360 nm or less; and the detached human cells are recovered together with the medium. If necessary, then, the medium was added thereto; the address regions of mouse cells were subjected to the scanning of a laser or light for photoreaction or the laser or light pattern irradiation; and the detached mouse cells were recovered together with the medium.

In this Example, in addition to the same advantages as those of Examples 5 and 6, in analyzing cells, individual cells can be caused to adhere to addresses in a lattice form to accelerate the speed of optical detection and analytic fractionation. Such a method enables ready fractionation while observing and analyzing the response and the like of cells to a compound, enabling real time handling.

### Example 10

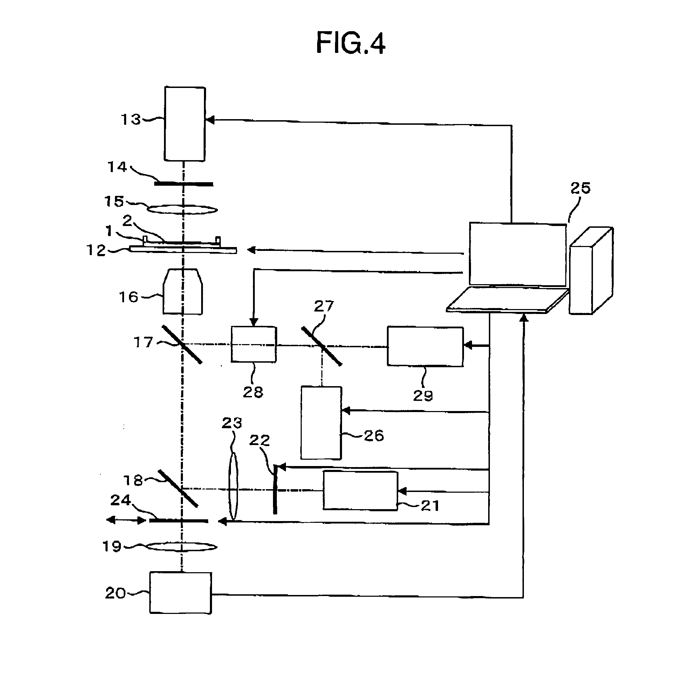

Fig. 4 shows an outline of one embodiment of the device for analyzing and fractionating cells according to the present invention.

In Fig. 4, the photo-controllable cell-adhesive substrate in which the photo-controllable cell-adhesive material 2 for adhering to cells is film-formed on the transparent base 1 is fixed on the stage 12 which can be motor and/or manually driven. The transparent base 1 and/or the stage 12 are engraved with a positional marker enabling the identification of their positions.

In microscopic observation, a lamp such as a halogen lamp having broad emission spectra is used as the light source 13 to condense light onto the substrate by the condenser lens 15 after cutting light of a wavelength of 450 nm or less with the wavelength filter 14. A transmitted light is condensed with the objective lens 16, passed through the two dichroic mirrors 17 and 18, condensed with the imaging lens 19, and detected with the (2-dimensional) detector 20 such as CCD. In fluorescent image observation, a xenon lamp, a high-pressure mercury lamp, or the like having broad emission spectra is used as the (excitation) light source 21, and light is passed through the single or the plurality of wavelength filters 22 for excitation wavelength selection, the collimator lens 23, and the dichroic mirrors 17 and 18, and then condensed onto the substrate with the objective lens 16. The generated fluorescence is condensed with the objective lens 16, passed through the dichroic mirrors 17 and 18, passed through the single or the plurality of wavelength filters 24 for cutting excitation light, condensed with the imaging lens 19, and detected with the (2-dimensional) detector 20 such as CCD. The detected data are sent to the control and analysis device 25 containing a monitor and an operating part; a characteristic amount of cells are extracted by the image analysis; and the positional information of the cells is obtained. The light source 21 uses a lamp in the above; however, it may also use a known laser light source such as an argon laser.

The second light irradiation means for ablation uses an ultraviolet laser of 355 nm or the like as the light source 26, and, after changing the optical path using the dichroic mirror 27, the laser light is scanned with the XY deflector 28 on the basis of the positional information of each of the above cells. The scanned laser light is guided to the objective lens 16 by the dichroic mirror 17 and irradiated on the substrate.

The first light irradiation means for photoreaction uses a semiconductor laser of 405 nm or the like as the light source 29, and, after passing through the dichroic mirror 27, the laser light is scanned with the XY deflector 28 on the basis of the positional information of each of the above cells. The scanned laser light is condensed onto the substrate by the objective lens 16 after changing the optical path using the dichroic mirror 17.

In this Example, the cells are held on the stage while being cultured. Thus, they do not disappear from the field of view as long as they do not move on the stage. Therefore, the lamp and the plurality of wavelength filters (a plurality of bandpass interference filters) can be used to sequentially switch the filters to enable a plurality of excitations. Thus, even the use of a plurality of fluorophores as markers eliminates the need for the use of many types of fluorescence excitation lasers.

When a plurality of fluorophores is used as markers, fluorescent images can be effectively detected by switching a plurality of wavelength filters. Bandpass interference filters having different wavelength ranges optimal for detecting the respective fluorescence intensities as wavelength filters is used, and switched in turns to detect a fluorescent image of each wavelength range. This enables the measurement of a plurality of labeled states with higher accuracy, and thereby enables discrimination and separation to be efficiently performed.

In this Example, because the first light irradiation means and the second light irradiation means do not simultaneously perform irradiation, a method of the common use of the XY deflector 28 is adopted. Thus, the laser light axes of the light sources 26 and 29 are made coaxial by the dichroic mirror 27. The on and off of the laser is controlled by the control and analysis device 25.

In the above example, the dichroic mirror 27 is set to characteristics such as 355 nm reflection and 405 nm transmission; the dichroic mirror 17, 355 nm to 405 nm reflection and 450 nm or more transmission; and the dichroic mirror 18, 355 nm to 500 nm reflection and 520 nm or more transmission. These wavelength characteristics are varied depending on the laser wavelength, the fluorophore, and the like used.

In this Example, in addition to the advantages of Examples 1 to 9, fluorescence digital information for each cell as from a flow cytometer as well as image information as from a conventional fluorescent image analyzer can be obtained; thus, it can be conveniently used as information for fractionating cells.

### Example 11

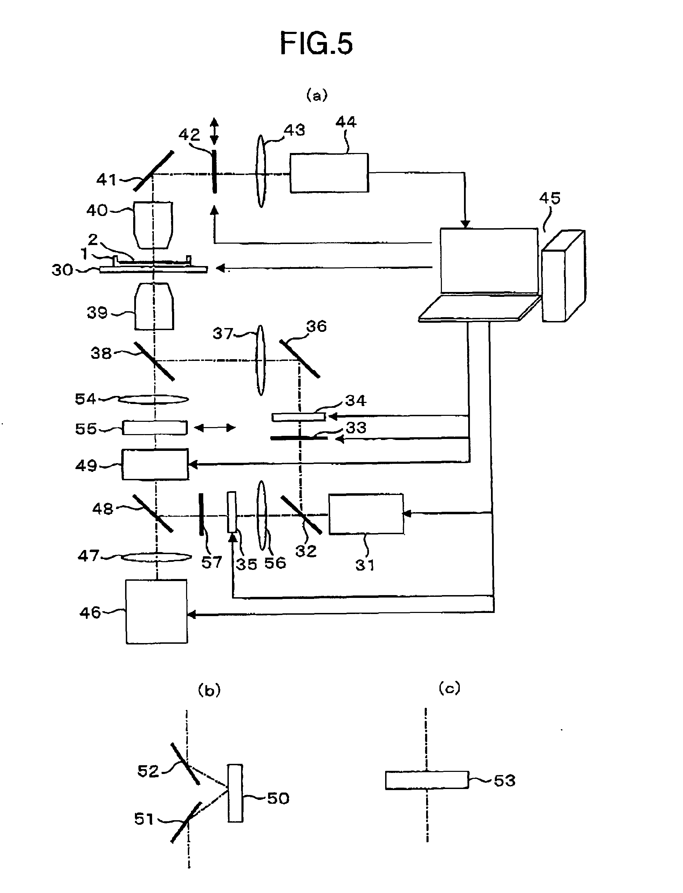

Fig. 5 shows an outline of one embodiment of the device for analyzing and fractionating cells according to the present invention.

In Fig. 5(a), the photo-controllable cell-adhesive substrate in which the photo-controllable cell-adhesive material 2 for adhering to cells is film-formed on the transparent base 1 is fixed on the stage 30 which can be motor and/or manually driven. The transparent base 1 and/or the stage 30 are engraved with a positional marker enabling the identification of their positions.

In the microscopic observation and/or the fluorescent observation, a lamp such as a xenon lamp or a high-pressure mercury lamp, having broad emission spectra is used as the light source 31; the light is divided into wavelengths by the dichroic mirror 32; and the reflected lights are each used as an illumination light for measuring a transmission image or an excitation light for measuring a fluorescent image. The transmitted lights from the dichroic mirror 32 are each used as an illumination light for the reaction of a photo-dissociable group. It is typically ensured that a sample is not simultaneously irradiated therewith.

The wavelengths of the reflected lights from the dichroic mirror 32 are selected by a plurality of the (light) wavelength filters 33 for transmitting light or selecting a fluorescence excitation wavelength. Then, the light is passed through the shutter 34 with the shutter 35 being closed. The light whose path is changed by the mirror 36 is passed through the collector lens 37, reflected by the dichroic mirror 38, guided to the objective lens 39, and condensed onto the substrate. The transmitted light or fluorescence is condensed by the objective lens 40, and, after the change of the light path by the mirror 41, transmitted light or a plurality of fluorescences are selected by a single or a plurality of wavelength filters 42, condensed by the imaging lens 43, and detected by the (2-dimensional) detector 44 such as CCD camera. The detected data are sent to the control and analysis device 45 containing a monitor and an operating part; a characteristic amount of cells are extracted by the image analysis; and the positional information of the cells is obtained.

The second light irradiation means for ablation uses a Nd:YAG near infrared laser of 1064 nm as the light source 46, and the light is made in the size of the pattern region by the collimator lens 47 and guided to the spatial light modulation device 49 through the dichroic mirror 48. The spatial light modulation device 49 may use the reflex spatial light modulation device 50 such as a digital mirror device shown in Fig. 5(b) or the liquid crystal spatial light modulation device 53 shown in Fig. 5(c). A mask pattern reflecting the positional information of cells is formed by the spatial light modulation device; a mask pattern is projected on the substrate through the relay lens 54, the dichroic mirror 38 and the objective lens 39; and light is irradiated on positions to be cut on the substrate. When the photo-controllable cell-adhesive substrate is larger than the region capable of being measured by and irradiated from the objective lens, the measurement and treatment is carried out by automatically or manually moving the stage 30 or by a step-and-repeat method for each region. When it is desired to set the ablation light wavelength in the ultraviolet region, the laser light source 46 for ablation uses, for example, a Nd:YAG near infrared laser of 1064 nm, which may be then used as a third harmonic having a 1/3 wavelength (355 nm) by placing the wavelength conversion device 55 such as a nonlinear crystal or a ferroelectric crystal between the spatial light modulation device 49 and the relay lens 54. For example, if a second harmonic is taken out using a visible ruby laser of 694 nm, it may be used as laser light of 347 nm.

The first light irradiation means for photoreaction is an optical system for condensing light onto the substrate through the spatial light modulation device 49 reflecting the positional information of each of the above cells. The light source 31 for photoreaction is in common with the light source for cell image detection. The light passing through the dichroic mirror 32 is guided to the spatial light modulation device 49 through the collector lens 56, the shutter 35, the wavelength filter 57, and the dichroic mirror 48. Here, the shutter 34 is closed. The wavelength used in this Example is adjusted, for example, to 360 to 450 nm by the wavelength filter 57. The spatial light modulation device 49 may use the reflex spatial light modulation device 50 such as a digital mirror device shown in Fig. 5(b) or the liquid crystal spatial light modulation device 53 shown in Fig. 5(c). A mask pattern reflecting the positional information of cells is formed by the spatial light modulation device; a desired mask pattern is projected on the substrate through the relay lens 54, the dichroic mirror 38 and the objective lens 39; and the surface characteristic of the intended regions is changed from a cell-adhesive one to a cell-non-adhesive one. This enables cells in the desired regions to be selectively detached and recovered. The position of the substrate can be changed through the stage 30 and wider portions can be treated by a step-and-repeat method.

This Example also has the same advantages as those of Example 10.

### Reference Signs List

- 1: Transparent Base
- 2: Photo-Controllable Cell-Adhesive Material
- 3, 4, 9: Cell
- 5: Second Light Irradiation
- 6: Cut Region between Cells and of Photo-Controllable Cell-Adhesive Material
- 7: First Light Irradiation
- 8: Region Changed from Cell-Adhesive One to Cell-Non-Adhesive One by Photoreaction (Cell-Non-Adhesive Region)
- 10, 11: Region Changed from Cell-Adhesive One to Cell-Non-Adhesive One by Photoreaction
- 12,30: Stage
- 13, 21: Light Source
- 14, 22, 24, 33, 42, 57: Wavelength Filter
- 15: Condenser Lens
- 16, 39, 40: Objective Lens
- 17, 18, 27, 32, 38, 48: Dichroic Mirror
- 19, 43: Imaging Lens
- 20,44: Detector
- 23, 47: Collimator Lens
- 25, 45: Control and Analysis Device Containing Monitor and Operating Part
- 26, 46: Second Light Irradiation Source
- 28: XY Deflector
- 29: First Light Irradiation Source
- 31: Light Source and First Light Irradiation Source
- 34, 35: Shutter
- 36,41,51,52: Mirror
- 37, 56: Collector Lens
- 49: Spatial Light Modulation Device
- 50: Reflex Spatial Light Modulation Device
- 53: Transmissive Spatial Light Modulation Device
- 54: Relay Lens
- 55: Wavelength Conversion Device
- 69: Cell-Adhesive Region

## Claims

1. A photo-controllable cell-adhesive substrate obtained by film-forming a photo-controllable cell-adhesive material in which a cell-adhesive material is bonded to a cell-non-adhesive material through a photo-dissociable group, on a base.

2. A photo-controllable cell-adhesive substrate, wherein light irradiation causes bond dissociation of a photo-dissociable group to produce separation of a cell-adhesive material to leave a cell-non-adhesive material.

3. A photo-controllable cell-adhesive substrate, wherein light irradiation causes bond dissociation of a photo-dissociable group to irreversibly change a surface of an irradiated portion from a cell-adhesive material to a cell-non-adhesive material.

4. The photo-controllable cell-adhesive substrate according to any of claims 1 to 3, wherein the cell-non-adhesive material is a material having a phosphorylcholine group.

5. The photo-controllable cell-adhesive substrate according to any of claims 1 to 3, wherein the cell-non-adhesive material comprises a (meth)acrylic ester polymer represented by general formula (1) below or a (meth)acrylic ester polymer represented by general formula (2) below or a copolymer of (meth)acrylic ester polymers represented by (1) and (2): 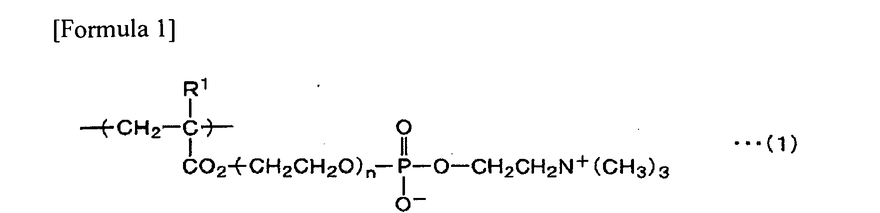 wherein R¹ represents hydrogen or a methyl group and n represents a number of 1 to 20; and 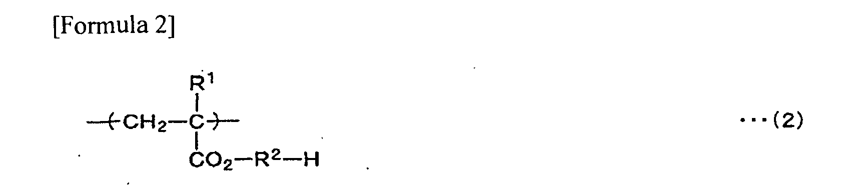 wherein R² represents 1 to 20 alkylene groups or 1 to 20 polyoxyethylene groups.

6. The photo-controllable cell-adhesive substrate according to any of claims 1 to 3, wherein the cell-non-adhesive material is an alkoxysilane represented by general formula (3) below:
[Formula 3] (R³O)₃Si—R²—H (3)
wherein R³ represents hydrogen or an alkyl group.

7. The photo-controllable cell-adhesive substrate according to any of claims 1 to 3, wherein the cell-adhesive material has a cell-adhesive group in a terminal end.

8. The photo-controllable cell-adhesive substrate according to any of claims 1 to 3, wherein the cell-adhesive material has a cell-adhesive group X represented by general formula (4) below in a terminal end:
[Formula 4] — X (4)
wherein X represents a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, or a nucleic acid group.

9. The photo-controllable cell-adhesive substrate according to any of claims 1 to 3, wherein the cell-adhesive material is a material in which an extracellular matrix capable of promoting adhesion to cells or an antibody capable of binding to a surface antigen of cells and a protein or the like for causing the antibody to bind thereto is bound or adheres to the cell-adhesive group.

10. The photo-controllable cell-adhesive substrate according to claim 9, wherein the extracellular matrix is a material selected from collagens, non-collagenous glycoproteins (fibronectin, vitronectin, laminin, nidogen, teneinosine, thrombospondi, von Willebrand, osteopontin, fibrinogen, and the like), elastins, and proteoglycans.

11. The photo-controllable cell-adhesive substrate according to claim 9, wherein the protein for causing the antibody to bind thereto is a material selected from avidin/biotin, protein A, or protein G.

12. The photo-controllable cell-adhesive substrate according to any of claims 1 to 3, wherein the photo-dissociable group has reactivity to light of a wavelength of 360 nm or more and shorter than a wavelength of incident light for observation or exciting light for fluorescent observation.

13. The photo-controllable cell-adhesive substrate according to any of claims 1 to 3, wherein the photo-dissociable group has reactivity to light at 360 nm to 450 nm.

14. The photo-controllable cell-adhesive substrate according to any of claims 1 to 3, wherein the photo-dissociable group comprises a divalent coumarinylmethyl skeleton.

15. The photo-controllable cell-adhesive substrate according to any of claims 1 to 3, wherein the photo-dissociable group comprises a divalent coumarinylmethyl skeleton represented by general formula (5) below: 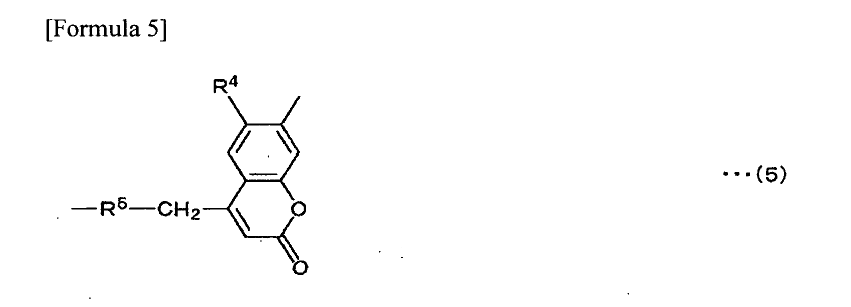 wherein R⁴ represents hydrogen or an alkoxy group and R⁵ is divalent and represents O, CO, CO₂, OCO₂, NHCO₂, NH, SO₃, or (OPO(OH))_{1 to 3} O.

16. The photo-controllable cell-adhesive substrate according to any of claims 1 to 3, wherein the photo-controllable cell-adhesive material comprises a structure in which a cell-adhesive group represented by general formula (6) directly or indirectly bonds to a photo-dissociable group represented by general formula (7) at position 7 of a coumarin skeleton thereof or at a position of R⁵:
[Formula 6] — X (6)

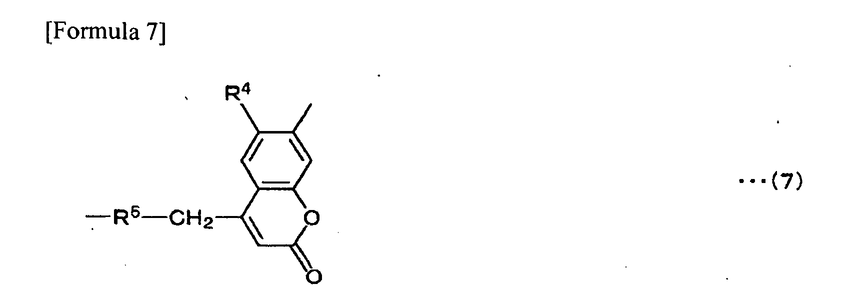

17. The photo-controllable cell-adhesive substrate according to any of claims 1 to 3, wherein the photo-controllable cell-adhesive material comprises a structure in which a photo-dissociable group represented by general formula (8) below bonds to a cell-adhesive group via a divalent linking group R⁶ at position 7 of a coumarin skeleton thereof:

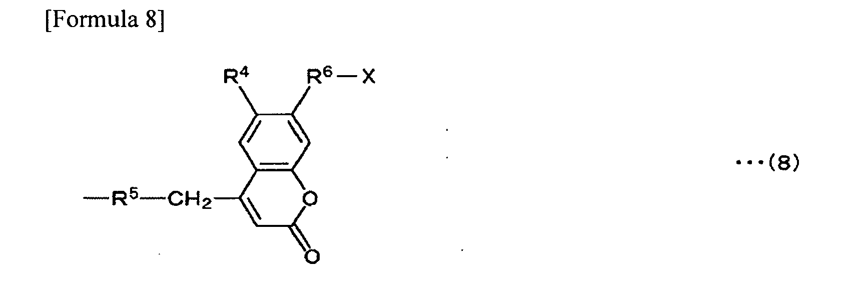

18. The photo-controllable cell-adhesive substrate according to any of claims 1 to 3, wherein the photo-controllable cell-adhesive material comprises a structure in which a photo-dissociable group represented by general formula (9) below bonds to a cell-adhesive group at a position of R⁵:

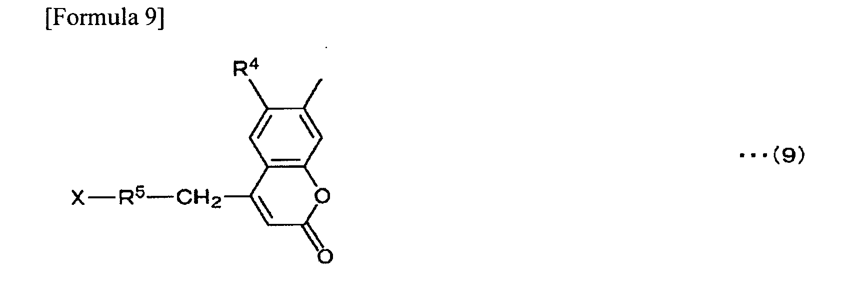

19. The photo-controllable cell-adhesive substrate according to claim 17, wherein the divalent linking group R⁶ is represented by any of O(CH₂)ₘ, O(CH₂CH₂O)ₘ, OCO(CH₂)ₘ, and OCOCH₂O(CH₂CH₂O)ₘ, wherein m represents an integer of 0 to 20.

20. The photo-controllable cell-adhesive substrate according to any of claims 1 to 3, wherein the photo-controllable cell-adhesive material comprises a structure in which a structure in which a cell-adhesive group represented by general formula (10) directly or indirectly bonds to a photo-dissociable group represented by general formula (11) at position 7 of a coumarin skeleton thereof or at a position of R⁵ directly or indirectly bonds to the cell-non-adhesive material:
[Formula 10] — X (10)

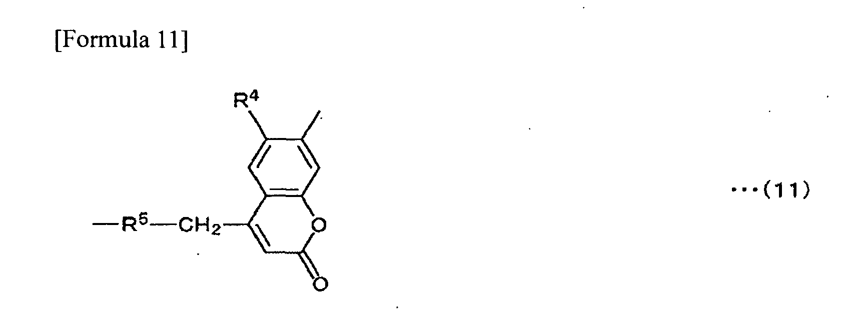

21. The photo-controllable cell-adhesive substrate according to any of claims 1 to 3, wherein the photo-controllable cell-adhesive material comprises a structure represented by general formula (12) below:

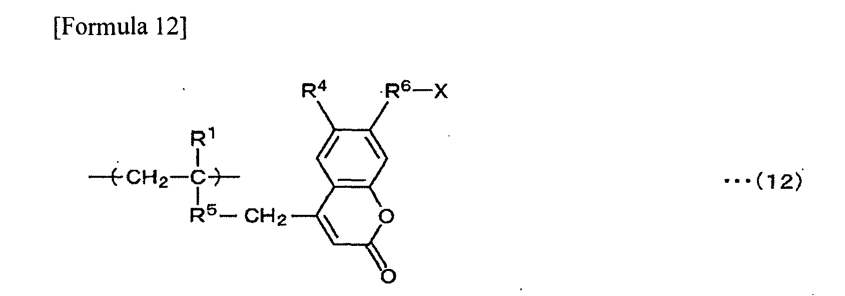

22. The photo-controllable cell-adhesive substrate according to any of claims 1 to 3, wherein the photo-controllable cell-adhesive material comprises a structure represented by general formula (13) below:

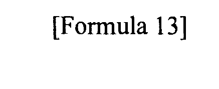

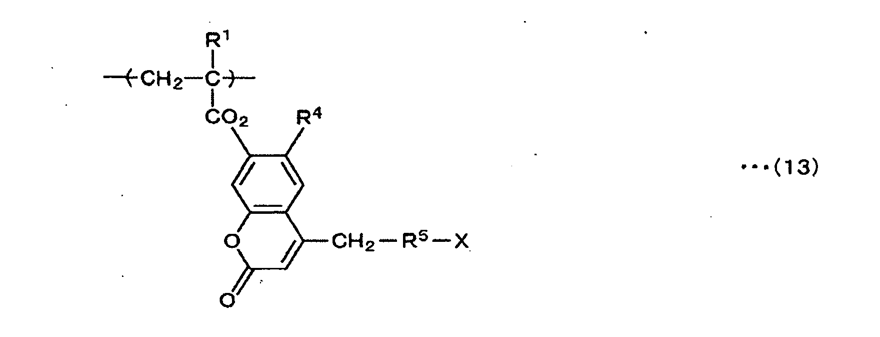

23. The photo-controllable cell-adhesive substrate according to any of claims 1 to 3, wherein the photo-controllable cell-adhesive material comprises a (meth)acrylic ester represented by general formula (14) below:

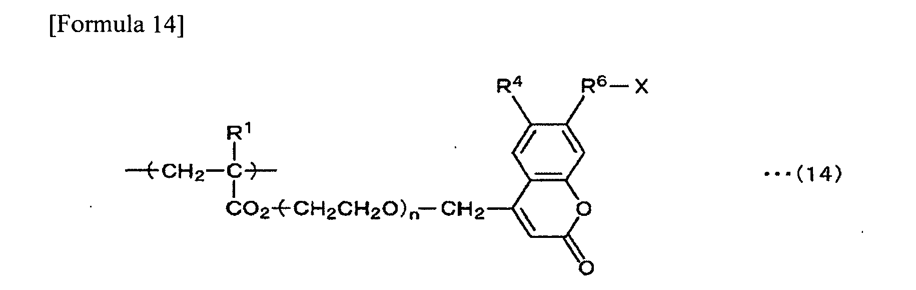

24. The photo-controllable cell-adhesive substrate according to any of claims 1 to 3, wherein the photo-controllable cell-adhesive material comprises a copolymer of (meth)acrylic ester polymers represented by general formulas (15) and (16) below, general formulas (15) and (17) below, general formulas (15) and (18) below, general formulas (15), (16), and (19) below, general formulas (15), (17), and (19) below, or general formulas (15), (18), and (19) below:

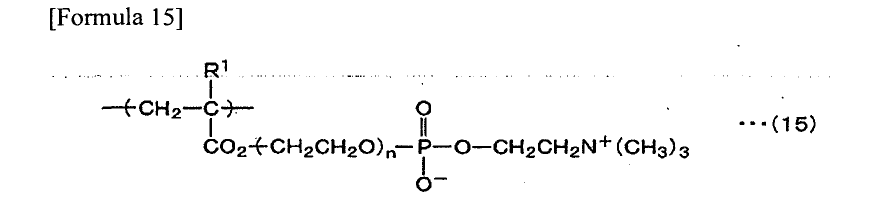

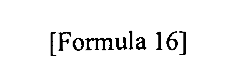

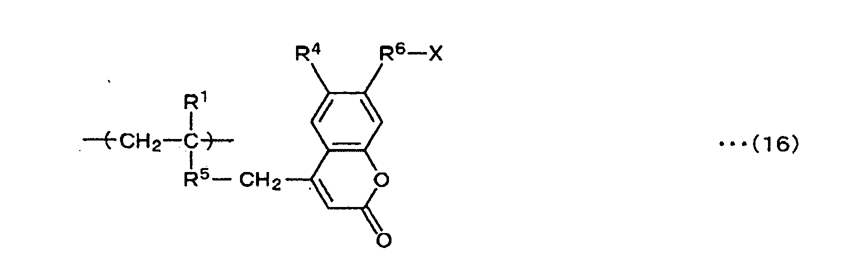

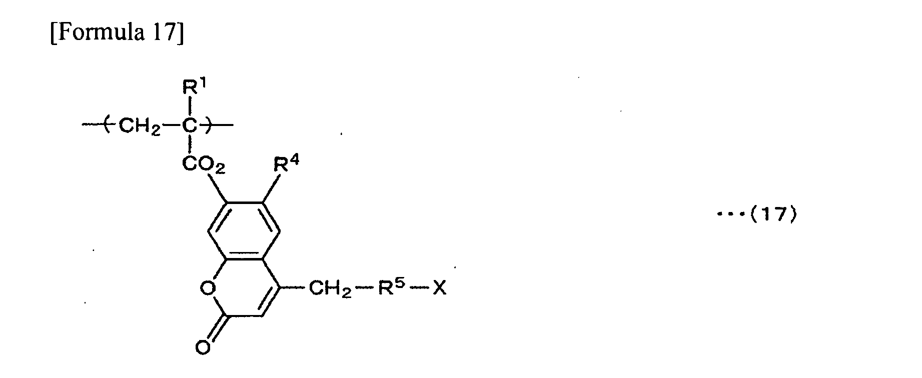

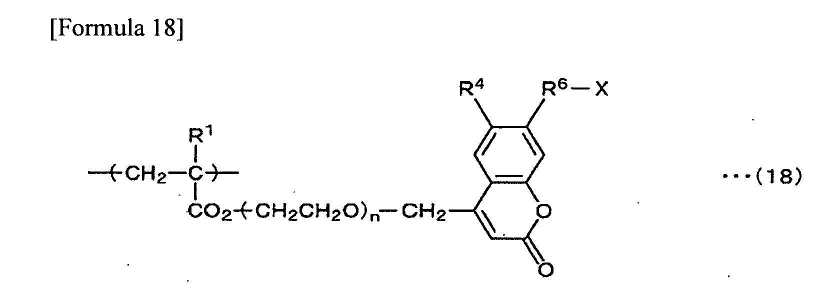

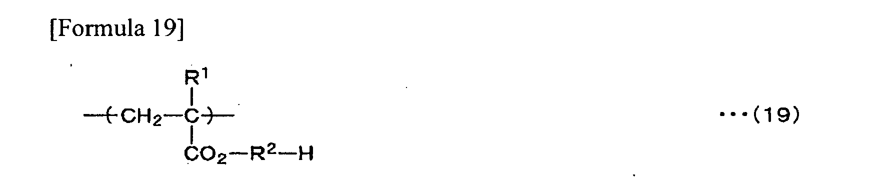

25. The photo-controllable cell-adhesive substrate according to any of claims 1 to 3, wherein the photo-controllable cell-adhesive material is obtained by copolymerizing a (meth)acrylic ester comprising an alkoxysilane in a side chain thereof.

26. The photo-controllable cell-adhesive substrate according to any of claims 1 to 3, wherein the photo-controllable cell-adhesive material is an alkoxysilane represented by general formula (20) below:

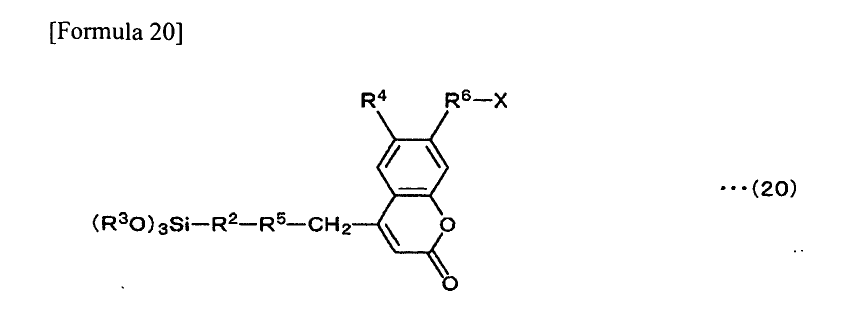

27. The photo-controllable cell-adhesive substrate according to any of claims 1 to 3, wherein the photo-controllable cell-adhesive material is an alkoxysilane represented by general formula (21) below:

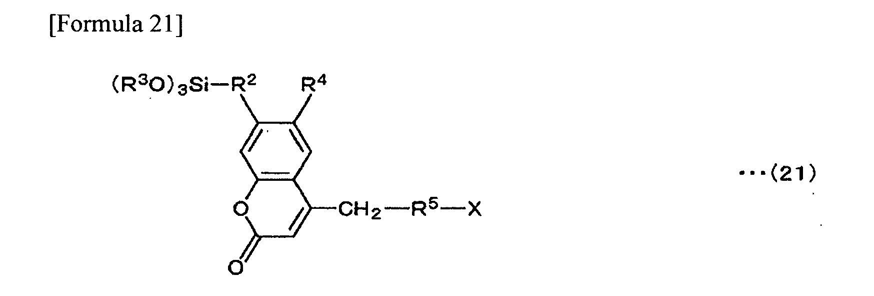

28. The photo-controllable cell-adhesive substrate according to any of claims 1 to 3, wherein the base is a glass culture vessel.

29. A method for analyzing and fractionating cells, comprising the steps of:
seeding and culturing cells in a photo-controllable cell-adhesive substrate obtained by film-forming a photo-controllable cell-adhesive material in which a cell-adhesive material is bonded to a cell-non-adhesive material through a photo-dissociable group, on a base, or a photo-controllable cell-adhesive substrate, wherein light irradiation causes bond dissociation of the photo-dissociable group to produce separation of the cell-adhesive material to leave the cell-non-adhesive material, or a photo-controllable cell-adhesive substrate, wherein light irradiation causes the bond dissociation of the photo-dissociable group to irreversibly change the surface of an irradiated portion thereof from the cell-adhesive material to a cell-non-adhesive material; and
detaching and recovering desired cells from the substrate by first light irradiation on desired cellular regions.

30. The method for analyzing and fractionating cells according to claim 29, further comprising the steps of:
detecting cell images and extracting a characteristic amount of cells; and
obtaining the positional information of desired cells.

31. The method for analyzing and fractionating cells according to claim 29, further comprising the step of:
cutting or destructing between desired cells and other cells and the photo-controllable cell-adhesive material by second light irradiation different from the first light irradiation.

32. A method for analyzing and fractionating cells, comprising the step of:
providing cell-adhesive regions and a cell-non-adhesive region by first light irradiation on a photo-controllable cell-adhesive substrate obtained by film-forming a photo-controllable cell-adhesive material in which a cell-adhesive material is bonded to a cell-non-adhesive material through a photo-dissociable group, on a base, or a photo-controllable cell-adhesive substrate, wherein light irradiation causes the bond dissociation of the photo-dissociable group to produce the separation of the cell-adhesive material to leave the cell-non-adhesive material, or a photo-controllable cell-adhesive substrate, wherein light irradiation causes the bond dissociation of the photo-dissociable group to irreversibly change the surface of the irradiated portion thereof from the cell-adhesive material to the cell-non-adhesive material.

33. The method for analyzing and fractionating cells according to claim 32, comprising the steps of:
seeding cells on the photo-controllable cell-adhesive substrate after the first light irradiation;
detecting cell images and extracting a characteristic amount of cells;
obtaining the positional information of desired cells;
performing the first light irradiation on cell-adhesive regions to which cells do not adhere to make the region cell-non-adhesive; and
detaching and recovering desired cells from the substrate by the first light irradiation on a desired cellular region.

34. The method for analyzing and fractionating cells according to claim 33, comprising the step of:
cutting or destructing the photo-controllable cell-adhesive material by the second light irradiation different from the first light irradiation to provide cell-adhesive regions and cell-non-adhesive regions.

35. The method for analyzing and fractionating cells according to claim 31 or 34, wherein the second light irradiation uses laser light.

36. The method for analyzing and fractionating cells according to claim 33 or 34, wherein the cell-adhesive regions have areas of single cells arranged in a lattice form and the cells in the step of seeding the cells are individually separated.

37. A device for analyzing and fractionating cells, comprising a photo-controllable cell-adhesive substrate obtained by film-forming a photo-controllable cell-adhesive material in which a cell-adhesive material is bonded to a cell-non-adhesive material through a photo-dissociable group, on a base, or a photo-controllable cell-adhesive substrate, wherein light irradiation causes bond dissociation of the photo-dissociable group to produce separation of the cell-adhesive material to leave the cell-non-adhesive material, or a photo-controllable cell-adhesive substrate, wherein light irradiation causes the bond dissociation of the photo-dissociable group to irreversibly change a surface of an irradiated portion thereof from the cell-adhesive material to a cell-non-adhesive material, and a first light irradiation means for subjecting the photo-controllable cell-adhesive material on the base to photoreaction.

38. The device for analyzing and fractionating cells according to claim 37, comprising a stage on which the substrate is placed, an optical detection means for obtaining cell images, a positional information acquisition means for obtaining positional information from cell images, and a means for controlling motion of each means.

39. The device for analyzing and fractionating cells according to claim 38, further comprising a second light irradiation means for cutting or destructing between cells and the photo-controllable cell-adhesive material.

40. The device for analyzing and fractionating cells according to claim 38, wherein a lamp or LED having broad emission spectra is used as a light source of the optical detection means and a wavelength of not more than a wavelength of the photoreaction of the photo-controllable cell-adhesive material or shorter than a fluorescence excitation wavelength (the photoreaction wavelength < the fluorescence excitation wavelength) is cut.

41. The device for analyzing and fractionating cells according to claim 38, wherein the light source of the optical detection means uses a laser of a wavelength longer than the photoreaction wavelength.

42. The device for analyzing and fractionating cells according to claim 38, wherein the optical detection means uses a 2-dimensional CCD camera or a photomultiplier tube as a detector.

43. The device for analyzing and fractionating cells according to claim 38, wherein the optical detection means performs detection using a line sensor through a dispersive element.

44. The device for analyzing and fractionating cells according to claim 39, wherein the second light irradiation means is an optical system for performing laser scanning by an XY deflector on the basis of positional information from the positional information acquisition means using a infrared laser or an ultraviolet laser as a light source.

45. The device for analyzing and fractionating cells according to claim 39, wherein the second light irradiation means is an optical system for condensing laser light onto the substrate through a spatial light modulation device reflecting positional information from the positional information acquisition means using a near infrared laser as a light source.

46. The device for analyzing and fractionating cells according to claim 39, wherein the second light irradiation means is an optical system for condensing laser light onto the substrate through the spatial light modulation device reflecting positional information from the positional information acquisition means and a wavelength conversion device using a laser in the visible to near infrared region as a light source.

47. The device for analyzing and fractionating cells according to claim 37, wherein the light source of the first light irradiation means uses a wavelength of photoreaction of the photo-controllable cell-adhesive material and employs a lamp or LED having broad emission spectra and a wavelength of 360 nm or less and, in some cases, not less than the fluorescence excitation wavelength is cut by a wavelength filter.

48. The device for analyzing and fractionating cells according to claim 37, wherein the light source of the first light irradiation means is a laser light source in a photoreaction wavelength region.

49. The device for analyzing and fractionating cells according to claim 37, wherein the first light irradiation means is an optical system for performing laser or light scanning by an XY deflector or an XY scanner on the basis of the positional information from the positional information acquisition means.

50. The device for analyzing and fractionating cells according to claim 37, wherein the first light irradiation means is an optical system for condensing light onto the substrate through the spatial light modulation device reflecting the positional information from the positional information acquisition means.

51. A device for analyzing and fractionating cells, wherein the spatial light modulation device according to claim 45, 46, or 50 is a reflex or transmissive spatial light modulation device.

52. A device for analyzing and fractionating cells, wherein the reflex or transmissive spatial light modulation device according to claim 51 is a digital mirror device or a liquid crystal spatial light modulation device.

53. A device for analyzing and fractionating cells, wherein the wavelength conversion device according to claim 46 is a nonlinear crystal or a ferroelectric crystal.
